# EUROPEAN PATENT SPECIFICATION

(11) **EP 2 834 255 B1**
(45) Date of publication and mention of the grant of the patent: **04.11.2020**
(21) Application number: 13769274.5
(22) Date of filing: 14.03.2013
(51) Int. Cl.: A61K 36/81, A61K 31/385, A61K 9/00

(54) **METHODS AND MATERIALS RELATED TO NUTRITIONAL SUPPLEMENT COMPOSITIONS CONTAINING A POTATO POLYSACCHARIDE PREPARATION**
VERFAHREN UND MATERIALIEN IN VERBINDUNG MIT NAHRUNGSERGÄNZUNGSZUSAMMENSETZUNGEN MIT EINER POLYSACCHARIDZUBEREITUNG AUS KARTOFFEL
PROCÉDÉS ET MATÉRIAUX SE RAPPORTANT À DES COMPOSITIONS DE SUPPLÉMENT NUTRITIF CONTENANT UNE PRÉPARATION DE POLYSACCHARIDES DE POMME DE TERRE

(30) Priority: 28.03.2012 US 201261616924 P
(43) Date of publication of application: 11.02.2015
(73) Proprietor: The Research Foundation for The State University of New York, Albany, NY 12207-2826 (US)
(72) Inventor: STEFANO, George B., Melville, New York 11747 (US); KREAM, Richard, Huntington, New York 11743 (US); MANTIONE, Kirk J., Patchogue New York 11772 (US)
(74) Representative: Fish & Richardson P.C.
(86) International application number: PCT/US2013/031700
(87) International publication number: WO 2013/148282

(56) References cited:
- WO-A1-2011/069781
- US-A1- 2004 038 933
- US-A1- 2008 213 400
- US-A1- 2008 279 984
- US-A1- 2011 081 475
- US-B2- 7 590 493
- NANDITA SINGH ET AL: "Protective Effect of Potato Peel Powder in Ameliorating Oxidative Stress in Streptozotocin Diabetic Rats", PLANT FOODS FOR HUMAN NUTRITION, KLUWER ACADEMIC PUBLISHERS, DO, vol. 60, no. 2, 1 June 2005 (2005-06-01), pages 49-54, XP019264485, ISSN: 1573-9104
- LUDVIK BERNHARD ET AL: "Efficacy of Ipomoea batatas (Caiapo) on diabetes control in type 2 diabetic subjects treated with diet", DIABETES CARE FEB 2004, vol. 27, no. 2, February 2004 (2004-02), pages 436-440, XP002751436, ISSN: 0149-5992
- JU JAE-HYUN ET AL: "Anti-obesity and antioxidative effects of purple sweet potato extract in 3T3-L1 adipocytes in vitro", JOURNAL OF MEDICINAL FOOD OCT 2011, vol. 14, no. 10, October 2011 (2011-10), pages 1097-1106, XP002751437, ISSN: 1557-7600
- KUSANO S ET AL: "Antidiabetic activity of white skinned sweet potato (Ipomoea batatas L.) in obese Zucker fatty rats", BIOLOGICAL & PHARMACEUTICAL BULLETIN JAN 2000, vol. 23, no. 1, January 2000 (2000-01), pages 23-26, XP002751438, ISSN: 0918-6158
- NAKAJIMA SHINGO ET AL: "Potato extract (Potein) suppresses food intake in rats through inhibition of luminal trypsin activity and direct stimulation of cholecystokinin secretion from enteroendocrine cells", JOURNAL OF AGRICULTURAL AND FOOD CHEMISTRY 14 SEP 2011, vol. 59, no. 17, 14 September 2011 (2011-09-14), pages 9491-9496, XP002751439, ISSN: 1520-5118
- KAZUHIKO YAMATOYA ET AL: "Hypolipidemic Effects of Hydrolyzed Xyloglucan", MACROMOLECULAR SYMPOSIA, vol. 120, no. 1, July 1997 (1997-07), pages 231-236, XP002751461, ISSN: 1022-1360, DOI: 10.1002/masy.19971200123
- TAN ET AL.: 'Regulation of mammalian pyruvate dehydrogenase alpha subunit gene expression by glucose in HepG2 cells.' BIOCHEM. J. vol. 336, no. PT 1, 15 November 1998, pages 49 - 56, XP002289821
- JARVIS M C ET AL: "The polysaccharide structure of potato cell walls: Chemical fractionation", PLANTA, SPRINGER VERLAG, DE, vol. 152, no. 2, 31 May 1981 (1981-05-31), pages 93-100, XP009513764, ISSN: 0032-0935, DOI: 10.1007/BF00391179

## Description

### BACKGROUND

### 1. Technical Field

The document relates to nutritional supplement compositions. For example, this document relates to nutritional supplement compositions containing a potato polysaccharide preparation,for increasing or decreasing expression of polypeptides involved with mitochondria activity or function.

### 2. Background Information

Potatoes are starchy, edible tubers obtained from potato plants and form an integral part of much of the world's food supply. In fact, potatoes are the fourth largest food crop in the world. The main potato species worldwide is *Solanum tuberosum.*

Nandita Singh et al. describes in Plant Foods For Human Nutrition vol. 60, no. 2, 1 June 2005, pages 49-54 the efficacy of *Ipomoea batatas* (Caiapo) on diabetes control in type 2 diabetic subjects treated with diet.

### SUMMARY

The present invention is defined by independent claim 1. The dependent claims depict other embodiments of the invention.

The document provides nutritional supplement compositions containing a potato polysaccharide preparation obtained from raw potatos. The potato polysaccharide preparation comprises at least 3,4-Furandimethanol,diacetate, 1,2,3,4,5-penta-o-acetyl-D-xylitol, 3,5-diacetoxybenzylalchohol and D-glucitol-hexaacetate. This document provides nutritional supplement compositions containing a potato polysaccharide preparation, methods for obtaining potato polysaccharide preparations, methods for making nutritional supplement compositions containing a potato polysaccharide preparation, and methods for increasing or decreasing expression of polypeptides involved with mitochondria activity or function The nutritional supplement compositions and potato polysaccharide preparations provided herein can be used to increase or decrease expression of polypeptides involved with mitochondria activity or function. For example, a nutritional supplement composition containing a potato polysaccharide preparation provided herein or a potato polysaccharide preparation provided herein can be used to increase expression of a transcription factor A, mitochondrial polypeptide (a TFAM polypeptide), an ATP synthase, H⁺ transporting, mitochondrial F1 complex, alpha subunit 1 polypeptide (an ATP5A1 polypeptide), a pyruvate dehydrogenase (lipoamide) alpha 1 polypeptide (a PDHA1 polypeptide), a pyruvate dehydrogenase (lipoamide) alpha 2 polypeptide (a PDHA2 polypeptide), a thimet oligopeptidase 1 polypeptide (a THOP1 polypeptide), or a combination thereof. The nutritional supplement composition containing a potato polysaccharide preparation provided herein or a potato polysaccharide preparation provided herein can be used to decrease expression of a forkhead box O1 polypeptide (a FOX01A polypeptide), a nuclear factor of kappa light polypeptide gene enhancer in B-cells 1 polypeptide (a NFKB1 polypeptide), a pyruvate dehydrogenase kinase, isozyme 2 polypeptide (a PDK2 polypeptide), a pyruvate dehydrogenase kinase, isozyme 4 polypeptide (a PDK4 polypeptide), a 3-hydroxy-3-methylglutaryl-CoA reductase polypeptide (a HMGCR polypeptide)..

The composition can comprise between 1 mg and 100 mg of the potato polysaccharide preparation. The composition can comprise between 6 mg and 20 mg of the potato polysaccharide preparation. The composition can comprise between 1 mg and 100 mg of the potato polysaccharide component of the potato polysaccharide preparation. The composition can comprise between 6 mg and 20 mg of the potato polysaccharide component of the potato polysaccharide preparation. The composition can be in the form of a tablet. The composition comprises alpha lipoic acid or alpha tocopherol. The potato polysaccharide preparation is a preparation obtained from raw potatoes. The potato polysaccharide preparation can be in an amount that, when administered to a mammal, results in between 0.075 mg and 0.5 mg of the potato polysaccharide component of the potato polysaccharide preparation being administered to the mammal per kg of body weight of the mammal. At least about 80 percent of the potato polysaccharide preparation can be potato polysaccharide. At least about 90 percent of the potato polysaccharide preparation can be potato polysaccharide. At least about 95 percent of the potato polysaccharide preparation can be potato polysaccharide. The mammal can be a human.

This document features a nutritional supplement composition comprising, or consisting essentially of, a potato polysaccharide preparation obtained from raw potatoes. The composition can comprise between 1 mg and 100 mg of the potato polysaccharide preparation. The composition can comprise between 6 mg and 20 mg of the potato polysaccharide preparation. The composition can comprise between 1 mg and 100 mg of the potato polysaccharide component of the potato polysaccharide preparation. The composition can comprise between 6 mg and 20 mg of the potato polysaccharide component of the potato polysaccharide preparation. The composition can be in the form of a tablet. The composition can comprise alpha lipoic acid. The composition can comprise alpha tocopherol. The potato polysaccharide preparation can be in an amount that, when administered to a mammal, results in between 0.05 mg and 0.5 mg of the potato polysaccharide component of the potato polysaccharide preparation being administered to the mammal per kg of body weight of the mammal. The potato polysaccharide preparation can be in an amount that, when administered to a mammal, results in between 0.075 mg and 0.25 mg of the potato polysaccharide component of the potato polysaccharide preparation being administered to the mammal per kg of body weight of the mammal. The mammal can be a human. At least about 80 percent of the potato polysaccharide preparation can be potato polysaccharide. At least about 90 percent of the potato polysaccharide preparation can be potato polysaccharide. At least about 95 percent of the potato polysaccharide preparation can be potato polysaccharide.

Unless otherwise defined, all technical and scientific terms used herein have the same meaning as commonly understood by one of ordinary skill in the art to which this invention pertains. Although methods and materials similar or equivalent to those described herein can be used in the practice or testing of the present invention, suitable methods and materials are described below. In case of conflict between the present specification and references cited herein, the present specification, including definitions, will control. In addition, the materials, methods, and examples are illustrative only and not intended to be limiting.

Other features and advantages of the invention will be apparent from the following detailed description, and from the claims.

### DESCRIPTION OF DRAWINGS

Figure 1 is an HPLC chromatogram of a 10% ACN extract of raw potato (Russet Burbank).
Figure 2 is an HPLC chromatogram of collected and re-purified 3.5 minute peak material from a 10% ACN extract of raw potato shown in Figure 1.
Figure 3 is a representative real time PCR amplification plot for TFAM expression.
Figure 4 is an LC/MS trace of 3.5 minute HPLC peak material.
Figure 5 is a full NMR spectrum of 3.5 minute HPLC peak material.
Figure 6 is an expanded NMR spectrum of 3.5 minute HPLC peak material.
Figure 7 is a total ion chromatogram of derivatized carbohydrate fragments of 3.5 minute HPLC peak material obtained from raw potato Russet Burbank).
Figure 8 is a fragmentation pattern of diacetamide. The peak fragmentation pattern is in the top panel, the compound library fragmentation match is in the bottom panel, and an overlay of the two is in the center panel.
Figure 9 is a fragmentation pattern of 3-acetoxy pyridine. The peak fragmentation pattern is in the top panel, the compound library fragmentation match is in the bottom panel, and an overlay of the two is in the center panel.
Figure 10 is a fragmentation pattern of 3,4-furan dimethanol, diacetate. The peak fragmentation pattern is in the top panel, the compound library fragmentation match is in the bottom panel, and an overlay of the two is in the center panel.
Figure 11 is a fragmentation pattern of 1,2,3-propanetriol diacetate. The peak fragmentation pattern is in the top panel, the compound library fragmentation match is in the bottom panel, and an overlay of the two is in the center panel.
Figure 12 is a fragmentation pattern of imidazolc, 2-acctamino-5-mcthyl. The peak fragmentation pattern is in the top panel, the compound library fragmentation match is in the bottom panel, and an overlay of the two is in the center panel.
Figure 13 is a fragmentation pattern of 6,7-dihydro-5H-pyrrol[2,1,c][1,2,4] triazole-3-carboxylic acid. The peak fragmentation pattern is in the top panel, the compound library fragmentation match is in the bottom panel, and an overlay of the two is in the center panel.
Figure 14 is a fragmentation pattern of acetic acid, 1-(2-methyltetrazol-5-yl) ethenyl ester. The peak fragmentation pattern is in the top panel, the compound library fragmentation match is in the bottom panel, and an overlay of the two is in the center panel.
Figure 15 is a fragmentation pattern of 1,2,3,4-butanetriol, tetraacetate (isomer 1). The peak fragmentation pattern is in the top panel, the compound library fragmentation match is in the bottom panel, and an overlay of the two is in the center panel.
Figure 16 is a fragmentation pattern of 1,2,3,4-butanetriol, tetraacetate (isomer 2). The peak fragmentation pattern is in the top panel, the compound library fragmentation match is in the bottom panel, and an overlay of the two is in the center panel.
Figure 17 is a fragmentation pattern of pentaerythritol tetraacetate. The peak fragmentation pattern is in the top panel, the compound library fragmentation match is in the bottom panel, and an overlay of the two is in the center panel.
Figure 18 is a fragmentation pattern of 1,2,3,4,5-penta-o-acetyl-D-xylitol (isomer 1). The peak fragmentation pattern is in the top panel, the compound library fragmentation match is in the bottom panel, and an overlay of the two is in the center panel.
Figure 19 is a fragmentation pattern of 1,2,3,4,5-penta-o-acetyl-D-xylitol (isomer 2). The peak fragmentation pattern is in the top panel, the compound library fragmentation match is in the bottom panel, and an overlay of the two is in the center panel.
Figure 20 is a fragmentation pattern of 3,5-diacetoxy benzyl alcohol. The peak fragmentation pattern is in the top panel, the compound library fragmentation match is in the bottom panel, and an overlay of the two is in the center panel.
Figure 21 is a fragmentation pattern of β-D-galactopyranose, pentaacetate. The peak fragmentation pattern is in the top panel, the compound library fragmentation match is in the bottom panel, and an overlay of the two is in the center panel.
Figure 22 is a fragmentation pattern of D-mannitol hexaacetate. The peak fragmentation pattern is in the top panel, the compound library fragmentation match is in the bottom panel, and an overlay of the two is in the center panel.
Figure 23 is a fragmentation pattern of galacticol, hexaacetate. The peak fragmentation pattern is in the top panel, the compound library fragmentation match is in the bottom panel, and an overlay of the two is in the center panel.
Figure 24 is a fragmentation pattern of cyclohexane carboxylic acid, 1,2,4,5-tetrakis(acetoxy), (1α,3α,4α,5β)-(-). The peak fragmentation pattern is in the top panel, the compound library fragmentation match is in the bottom panel, and an overlay of the two is in the center panel.
Figure 25 is a fragmentation pattern of muco-inositol, hexaacetate. The peak fragmentation pattern is in the top panel, the compound library fragmentation match is in the bottom panel, and an overlay of the two is in the center panel.
Figure 26 is a fragmentation pattern of D-glucitol-hexaacetate. The peak fragmentation pattern is in the top panel, the compound library fragmentation match is in the bottom panel, and an overlay of the two is in the center panel.
Figure 27 is a fragmentation pattern of myo-inositol, hexaacetate. The peak fragmentation pattern is in the top panel, the compound library fragmentation match is in the bottom panel, and an overlay of the two is in the center panel.
Figure 28 is an IIPLC chromatogram of a 10% ACN extract of raw Organic Yellow potato.
Figure 29 is an HPLC chromatogram of a 10% ACN extract of raw Purple potato.
Figure 30 is an HPLC chromatogram of a 10% ACN extract of raw Idaho Russet potato.
Figure 31 is an HPLC chromatogram of a 10% ACN extract of raw Yukon Gold potato.
Figure 32 is an HPLC chromatogram of a 10% ACN extract of raw sweet potato.
Figure 33 is an HPLC chromatogram of a 10% ACN extract of boiled Purple potato.
Figure 34 is an HPLC chromatogram of two pooled fraction collections from Idaho Russet potatoes.
Figure 35 is an HPLC chromatogram of fractions collections from 3 g of purple potatoes.

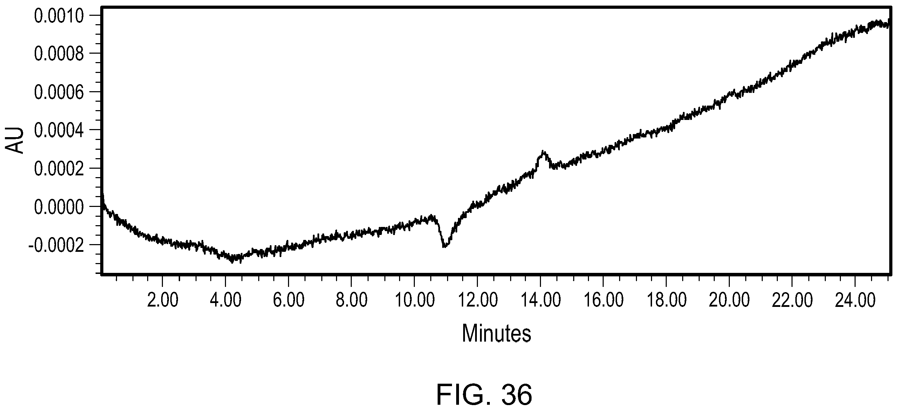

Figure 36 is an HPLC chromatogram of media collected from cells exposed to a potosaccharide preparation for 4 hours.

### DETAILED DESCRIPTION

The document provides nutritional supplement compositions. For example, this document provides nutritional supplement compositions containing a potato polysaccharide preparation, methods for obtaining potato polysaccharide preparations, methods for making nutritional supplement compositions containing a potato polysaccharide preparation, and methods and materials for increasing or decreasing expression of polypeptides involved with mitochondria activity or function.

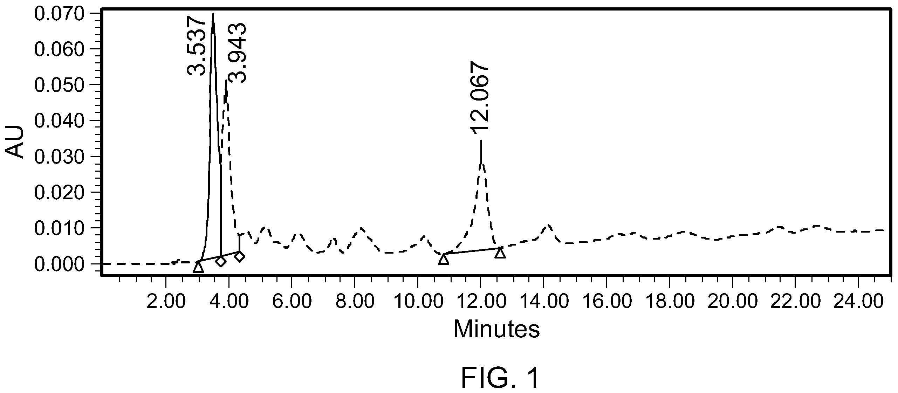

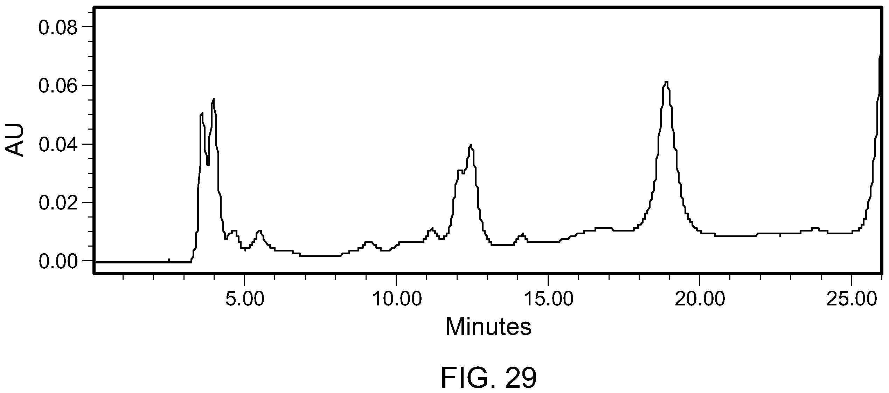

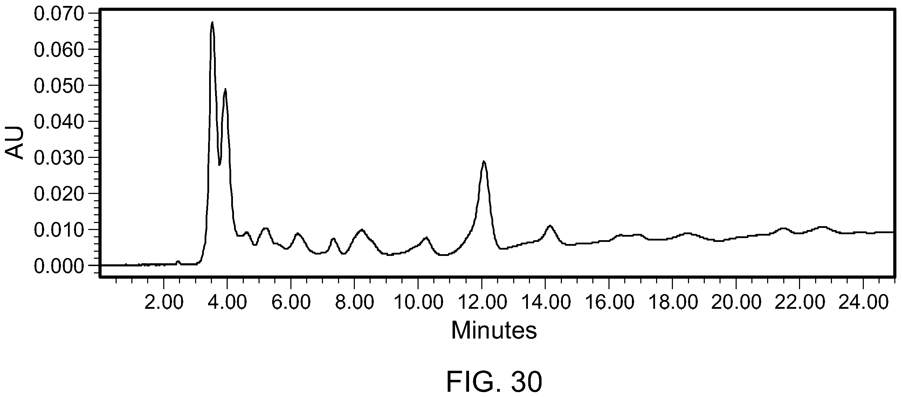

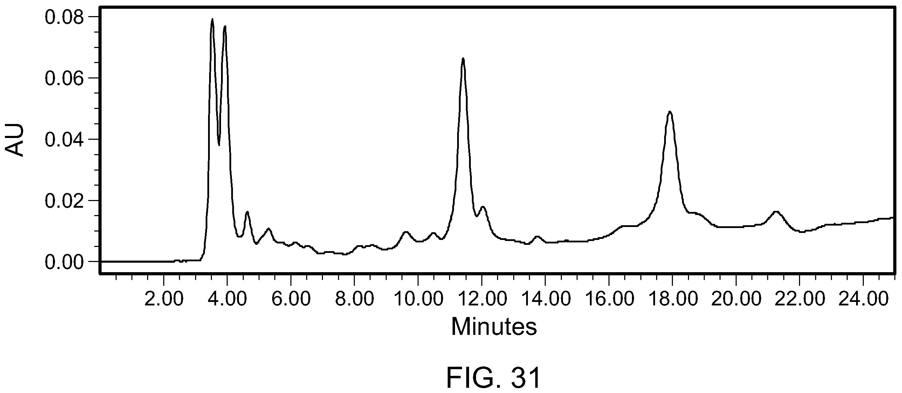

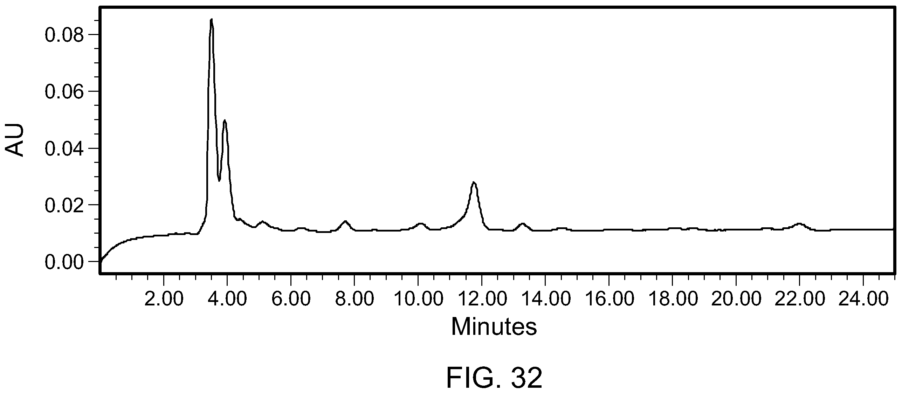

The nutritional supplement compositions provided herein can include one or more potato polysaccharide preparations. A potato polysaccharide preparation can be a preparation that is obtained from a water extract of potato and that contains polysaccharide material having the ability to be eluted from a C18 cartridge (e.g., a Sep-Pak Plus C-18 cartridge) with 10% acetonitrile. In some cases, a potato polysaccharide preparation can be a preparation that is obtained from potato and that contains polysaccharide material having HPLC characteristics of that of the peak eluted at 3.5 minutes as described in Example 1 (see, also, Figures 1, 2, and 28-34). In some cases, a polysaccharide of a potato polysaccharide preparation provided herein can be a polar, water-soluble polysaccharide. In some cases, a polysaccharide of a potato polysaccharide preparation provided herein can be a highly substituted complex xyloglucan material.

In some cases, a potato polysaccharide preparation can be a preparation that is obtained from potato and that contains polysaccharide material that, when derivatized, results in at least the following acylated carbohydrates as assessed using GC/MS: (a) myo-inositol (set to 1X to serve as an internal standard), (b) glucose at about 40X to about 60X the myo-inositol content (e.g., glucose at about 50X the myo-inositol content), (c) xylose at about 10X to about 20X the myo-inositol content (e.g., xylose at about 15X the myo-inositol content), (d) mannose at about 5X to about 15X the myo-inositol content (e.g., mannose at about 10X the myo-inositol content), and (e) galactose at about 3X to about 7X the myo-inositol content (e.g., galactose at about 5X the myo-inositol content). The derivatization procedure can include forming a dry residue of the polysaccharide material that is then hydrolyzed using trifluoroacetic acid. The resulting material is then reduced using sodium borohydride, and after borate removal, the end product is acylated using acetic anhydride and pyridine. The end products of the reaction are then injected directly on GC/MS to identify the acylated carbohydrates.

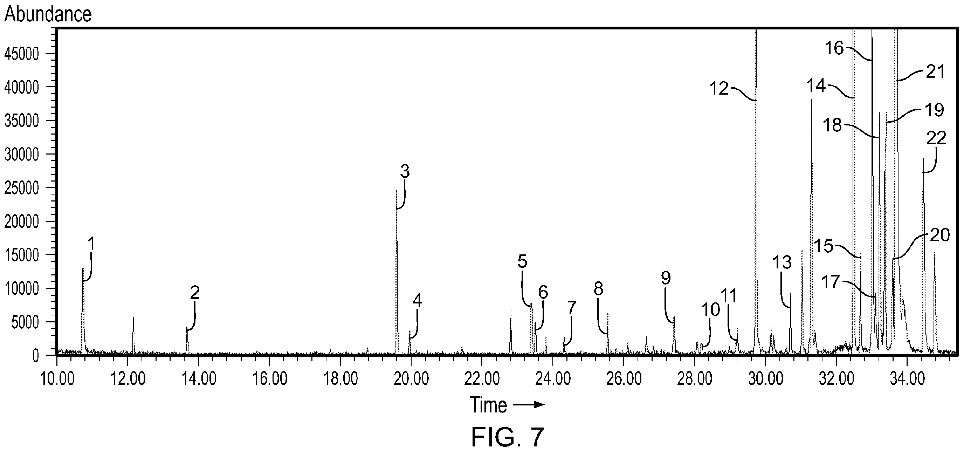

In some cases, a potato polysaccharide preparation can be a preparation that is obtained from potato and that contains polysaccharide material that, when derivatized and assessed using GC/MS, results in at least four major components (3,4-furan dimethanol, diacetate; 1,2,3,4,5-penta-o-acetyl-D-xylitol (isomer 1); 3,5-diacetoxy-benzyl alcohol; and D-glucitol-hexaacetate). See, e.g., Example 1. In some cases, a potato polysaccharide preparation can be a preparation that is obtained from potato and that contains polysaccharide material that, when derivatized and assessed using GC/MS, results in the compounds listed in Table 3 or results in the profile shown in Figure 7.

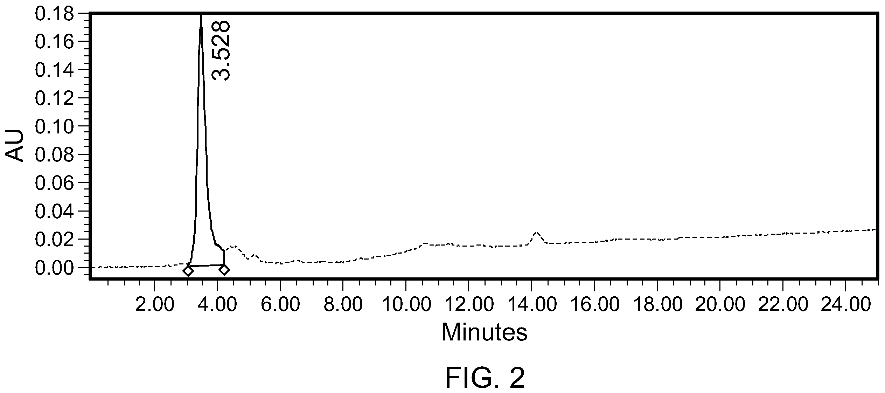

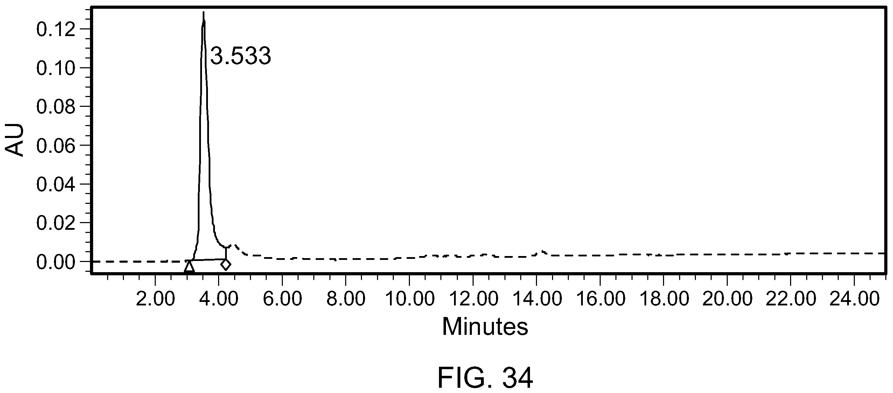

In some cases, a potato polysaccharide preparation provided herein can be a substantially pure potato polysaccharide preparation. Typically, a substantially pure potato polysaccharide preparation is a preparation that contains a single peak of material (e.g., a single peak of polysaccharide material) when assessed using, for example, HPLC (see, e.g., Figures 2 and 34). In some cases, greater than 60, 70, 75, 80, 85, 90, 95, or 99 percent of a potato polysaccharide preparation provided herein can be polysaccharide material obtained from a potato.

Any appropriate potato species or variety can be used to obtain a potato polysaccharide preparation provided herein. For example, *Solanum tuberosum, Ipomoea batatas, S. acaule, S. bukasovii, S. leptophyes, S. megistacrolobum, S. commersonii,* or *S. infundibuliforme* can be used to obtain a potato polysaccharide preparation provided herein. In some cases, potato varieties of *S*. *tunerosum* such as Organic Yellow, Purple or blue varieties, Cream of the Crop, Adirondack Blue, Adirondack Red, Agata, Almond, Andes Gold, Andes Sun, Apline, Alturas, Amandine, Annabelle, Anya, Arran Victory, Atlantic, Avalanche, Bamberg, Bannock Russet, Belle de Fontenay, BF-15, Bildtstar, Bintje, Blazer Russet, Blue Congo, Bonnotte, British Queens, Cabritas, Camota, Canela Russet, Cara, Carola, Chelina, Chiloé, Cielo, Clavela Blanca, Désirée, Estima, Fianna, Fingerling, Flava, German Butterball, Golden Wonder, Goldrush, Home Guard, Innovator, Irish Cobbler, Jersey Royal, Kennebec, Kerr's Pink, Kestrel, Keuka Gold, King Edward, Kipfler, Lady Balfour, Langlade, Linda, Marcy, Marfona, Maris Piper, Marquis, Megachip, Monalisa, Nicola, Pachacoña, Pike, Pink Eye, Pink Fir Apple, Primura, Ranger Russet, Ratte, Record, Red LaSoda, Red Norland, Red Pontiac, Rooster, Russet Burbank, Russet Norkotah, Selma, Shepody, Sieglinde, Silverton Russet, Sirco, Snowden, Spunta, Up to date, Stobrawa, Superior, Vivaldi, Vitelotte, Yellow Finn, or Yukon Gold can be used to obtain a potato polysaccharide preparation provided herein.

Any appropriate method can be used to obtain a potato polysaccharide preparation provided herein. For example, raw potato material can be homogenized (e.g., homogenized with a Polytron homogenizer) in water and maintained at room temperature for a period of time (e.g., about 1 hour) with occasional shaking. The homogenate can be centrifuged (e.g., centrifuged at 4000 g for 10 minutes) to remove any larger solid material. The resulting supernatant can be loaded onto a Solid Phase Extraction cartridge (e.g., a C18 cartridge such as a Sep-Pak Plus C-18 cartridge), and the polysaccharide material eluted with 10 percent acetonitrile. Once eluted, the polysaccharide material can be dried and stored (e.g., stored at about 4°C).

This document also provides nutritional supplement compositions containing one or more potato polysaccharide preparations provided herein. For example, a potato polysaccharide preparation provided herein obtained from Idaho Russet potatoes can be formulated into a nutritional supplement composition. Any appropriate dose of a potato polysaccharide preparation provided herein can be used to formulate a nutritional supplement composition. For example, a potato polysaccharide preparation provided herein can be used to formulate a nutritional supplement composition such that the nutritional supplement composition contains between about 1 mg and about 750 mg (e.g., between about 1 mg and about 500 mg, between about 1 mg and about 250 mg, between about 5 mg and about 40 mg, between about 5 mg and about 30 mg, between about 5 mg and about 20 mg, between about 6 mg and about 50 mg, between about 6 mg and about 20 mg, between about 10 mg and about 25 mg, or between about 15 mg and about 20 mg) of the potato polysaccharide component of the potato polysaccharide preparation. In some case, a nutritional supplement composition can be formulated to deliver about 0.05 mg of the potato polysaccharide component per kg of body weight to about 0.5 mg of the potato polysaccharide component per kg of body weight to a mammal (e.g., a human) per day. For example, such nutritional supplement compositions can be formulated into a single oral composition that a human can swallow once a day to provide between about 0.05 mg of the potato polysaccharide component per kg of body weight to about 0.5 mg of the potato polysaccharide component per kg of body weight.

Any appropriate method can be used to formulate a nutritional supplement composition provided herein. For example, common formulation mixing and preparation techniques can be used to make a nutritional supplement composition having the components described herein. In addition, a nutritional supplement composition provided herein can be in any form. For example, a nutritional supplement composition provided herein can be formulated into a pill, capsule, tablet, gelcap, nutritional shake, nutritional bar, rectal supository, sublingual suppository, nasal spray, inhalant, or injectable ampule. A nutritional supplement composition provided herein can include one or more potato polysaccharide preparations provided herein alone or in combination with other ingredients including, without limitation, gelatin, cellulose, starch, sugar, bentonite, lactic acid, mannitol, alpha lipoic acid, alpha tocopherol, L-ascorbate, or combinations thereof.

This document also provides methods for increasing or decreasing expression of polypeptides involved with mitochondria activity or function. For example, a potato polysaccharide preparation provided herein or a nutritional supplement composition provided herein can be used to increase or decrease expression of polypeptides involved with mitochondria activity or function. In some cases, a potato polysaccharide preparation provided herein or a nutritional supplement composition provided herein can be used to increase expression of a TFAM polypeptide, an ATP5A1 polypeptide, a PDHA1 polypeptide, a PDHA2 polypeptide, a THOP1 polypeptide, or a combination thereof. In some cases, a potato polysaccharide preparation provided herein or a nutritional supplement composition provided herein can be used to decrease expression of a F0X01A polypeptide, a NFKB1 polypeptide, a PDK2 polypeptide, a PDK4 polypeptide, a HMGCR polypeptide, or a combination thereof. In some case, a potato polysaccharide preparation provided herein or a nutritional supplement composition provided herein can be used to increase one or more polypeptides (e.g., one or more of a TFAM polypeptide, an ATP5A1 polypeptide, a PDHA1 polypeptide, a PDHA2 polypeptide, or a THOP1 polypeptide) and decrease one or more polypeptides (e.g., one or more of a FOX01A polypeptide, a NFKB1 polypeptide, a PDK2 polypeptide, a PDK4 polypeptide, or a HMGCR polypeptide).

In humans, a potato polysaccharide preparation provided herein or a nutritional supplement composition provided herein can be used to increase one or more human polypeptides (e.g., one or more of a human TFAM polypeptide, a human ATP5A1 polypeptide, a human PDHA1 polypeptide, a human PDHA2 polypeptide, a human THOP1 polypeptide, a human LIPE polypeptide (in adipocytes), a human PCK2 polypeptide, a human MOGAT1 polypeptide, a human PPARGC1a polypeptide, a vPPARGC1b polypeptide, an human SOD2 polypeptide, a human NR4A1 polypeptide (in adipocytes), a human ACAT2 polypeptide, or a human HMGCR polypeptide (in muscle cells)) and/or decrease one or more human polypeptides (e.g., one or more of a human FOX01A polypeptide, a human NFKB1 polypeptide, a human PDK2 polypeptide, a human PDK4 polypeptide, a human HMGCR polypeptide (in adipocytes), a human AGPAT1 polypeptide, a human OLR1 polypeptide, a human BCAT2 polypeptide, a human SH2B1 polypeptide, a human LPL polypeptide, a human HMGCR polypeptide (in adipocytes), a human LIPE polypeptide (in muscle cells), a human NR4A1 polypeptide (in muscle cells), a human PTEN polypeptide, or a human CASP8 polypeptide).

A human TFAM polypeptide can have the amino acid sequence set forth in GenBank® Accession No. CAG28581.1 (GI No. 47115243) and can be encoded by the nucleic acid sequence set forth in GenBank® Accession No. NM_003201.1 (GI No. 4507400). A human ATP5A1 polypeptide can have the amino acid sequence set forth in GenBank® Accession No.AAH08028.2 (GI No. 34782901) and can be encoded by the nucleic acid sequence set forth in GenBank® Accession No.NM_001001937.1 (GI No. 50345983). A human PDHA1 polypeptide can have the amino acid sequence set forth in GenBank® Accession No. ABQ58815.1 (GI No. 148300624) and can be encoded by the nucleic acid sequence set forth in GenBank® Accession No. NM_001173454.1 (GI No. 291084741). A human PDHA2 polypeptide can have the amino acid sequence set forth in GenBank® Accession No. AAH94760.1 (GI No. 66267554) and can be encoded by the nucleic acid sequence set forth in GenBank® Accession No. NM_005390.4 (GI No. 134031963). A human THOP1 polypeptide can have the amino acid sequence set forth in GenBank® Accession No. AAH00583.2 (GI No. 38014202) and can be encoded by the nucleic acid sequence set forth in GenBank® Accession No. NM_003249.3 (GI No. 34222291). A human LIPE polypeptide can have the amino acid sequence set forth in GenBank® Accession No. AAH70041.1 (GI No. 47124456) and can be encoded by the nucleic acid sequence set forth in GenBank® Accession No. NM_005357.2 (GI No. 21328445). A human PCK2 polypeptide can have the amino acid sequence set forth in GenBank® Accession No. CAG33194.1 (GI No. 48145943) and can be encoded by the nucleic acid sequence set forth in GenBank® Accession No. NM_004563.1 (GI No. 66346720). A human MOGAT1 polypeptide can have the amino acid sequence set forth in GenBank® Accession No. NP_477513.2 (GI No. 148746191) and can be encoded by the nucleic acid sequence set forth in GenBank® Accession No. NM_058165.1 (GI No. 148746190). A human PPARGC1a polypeptide can have the amino acid sequence set forth in GenBank® Accession No. NP_037393.1 (GI No. 7019499) and can be encoded by the nucleic acid sequence set forth in GenBank® Accession No. NM_013261.2 (GI No. 116284374). A human PPARGClb polypeptide can have the amino acid sequence set forth in GenBank® Accession No. AAI44252.1 (GI No. 219518198) and can be encoded by the nucleic acid sequence set forth in GenBank® Accession No. NM_133263.2 (GI No. 289577087). A human SOD2 polypeptide can have the amino acid sequence set forth in GenBank® Accession No. AAH16934.1 (GI No. 16877367) and can be encoded by the nucleic acid sequence set forth in GenBank® Accession No. NM_000636.1 (GI No. 67782304). A human NR4A1 polypeptide can have the amino acid sequence set forth in GenBank® Accession No. CAG32985.1 (GI No. 48145525) and can be encoded by the nucleic acid sequence set forth in GenBank® Accession No. NM_173158.1 (GI No. 320202954). A human ACAT2 polypeptide can have the amino acid sequence set forth in GenBank® Accession No. AAH00408.1 (GI No. 12653279) and can be encoded by the nucleic acid sequence set forth in GenBank® Accession No. NM_005891.1 (GI No. 148539871). A human FOX01A polypeptide can have the amino acid sequence set forth in GenBank® Accession No. NP_002006.2 (GI No. 9257222) and can be encoded by the nucleic acid sequence set forth in GenBank® Accession No. NM_002015.3 (GI No. 133930787). A human NFKB1 polypeptide can have the amino acid sequence set forth in GenBank® Accession No. CAB94757.1 (GI No. 8574070) and can be encoded by the nucleic acid sequence set forth in GenBank® Accession No. NM_001165412.1 (GI No. 25955301). A human PDK2 polypeptide can have the amino acid sequence set forth in GenBank® Accession No. NP_002602.2 (GI No. 19923736) and can be encoded by the nucleic acid sequence set forth in GenBank® Accession No. NM_00211.4 (GI No. 315630394). A human PDK4 polypeptide can have the amino acid sequence set forth in GenBank® Accession No. AAH40239.1 (GI No. 25955471) and can be encoded by the nucleic acid sequence set forth in GenBank® Accession No. NM_002612.2 (GI No. 94421466). A human HMGCR polypeptide can have the amino acid sequence set forth in GenBank® Accession No. AAH33692.1 (GI No.21707182) and can be encoded by the nucleic acid sequence set forth in GenBank® Accession No. NM_000859.2 (GI No. 196049378). A human AGPAT1 polypeptide can have the amino acid sequence set forth in GenBank® Accession No. NP_116130.2 (GI No. 15100175) and can be encoded by the nucleic acid sequence set forth in GenBank® Accession No. NM_006411.3 (GI No. 301336168). A human OLR1 polypeptide can have the amino acid sequence set forth in GenBank® Accession No. NP_002534.1 (GI No. 4505501) and can be encoded by the nucleic acid sequence set forth in GenBank® Accession No. NM_002543.2 (GI No. 119392084). A human BCAT2 polypeptide can have the amino acid sequence set forth in GenBank® Accession No. AAH04243.2 (GI No. 48257075) and can be encoded by the nucleic acid sequence set forth in GenBank® Accession No. NM_001190.1 (GI No. 258614013). A human SH2B1 polypeptide can have the amino acid sequence set forth in GenBank® Accession No. AAH10704.1 (GI No. 14715079) and can be encoded by the nucleic acid sequence set forth in GenBank® Accession No. NM_001145797.1 (GI No. 224926829). A human LPL polypeptide can have the amino acid sequence set forth in GenBank® Accession No. CAG33335.1 (GI No. 4814622) and can be encoded by the nucleic acid sequence set forth in GenBank® Accession No. NM_000237.1 (GI No. 145275217). A human HMGCR polypeptide can have the amino acid sequence set forth in GenBank® Accession No. AAH33692.1 (GI No. 21707182) and can be encoded by the nucleic acid sequence set forth in GenBank® Accession No. NM_001130996.1 (GI No. 196049379). A human PTEN polypeptide can have the amino acid sequence set forth in GenBank® Accession No. AAD13528.1 (GI No. 4240387) and can be encoded by the nucleic acid sequence set forth in GenBank® Accession No. NM_000314.2 (GI No. 110224474). A human CASP8 polypeptide can have the amino acid sequence set forth in GenBank® Accession No. AAH68050.1 (GI No. 45751586) and can be encoded by the nucleic acid sequence set forth in GenBank® Accession No. NM_001228.4 (GI No. 122056470).

The potato polysaccharide preparations provided herein or nutritional supplement compositions provided herein can be administered to any mammal (e.g., rat, mouse, dog, cat, horse, cow, goat, pig, chicken, duck, rabbit, sheep, monkey, or human). In addition, any route of administration (e.g., oral or parenteral administration) can be used to administer a potato polysaccharide preparation provided herein or a nutritional supplement composition provided herein to a mammal. For example, a potato polysaccharide preparation provided herein or a nutritional supplement composition provided herein can be administered orally.

The document will provide addition description in the following examples, which do not limit the scope of the invention described in the claims.

### EXAMPLES

### Example 1 - Identification of a potato polysaccharide preparation having the ability to alter expression of polypeptides involved with mitochondria activity and function

6 grams of a Russet potato variety of the *Solanum tuberosum* species were homogenized with a Polytron homogenizer in 20 mL water in a 50 mL centrifuge tube and kept at room temperature for 1 hour. The homogenate was centrifuged at 4000 rpm for 10 minutes. A Sep-Pak Plus C-18 cartridge was activated with 10 mL 100% acetonitrile (ACN) and washed with 10 mL 0.05% trifluoroacetic acid in water (TFA water). 10 mL of the supernatant was loaded onto the cartridge, and all H₂O that passes through cartridge was collected in 1.5 mL Eppendorf tubes. Next, 10 mL of 2% ACN (in 0.05%TFA water) was passed through the column, and the elutriate was collected in 1.5 mL Eppendorf tubes. Next, 10 mL of 5% ACN (in 0.05%TFA water) was used to wash the column, and the elutriate was collected in 1.5 mL Eppendorf tubes. Finally, 10 mL of 10% ACN (in 0.05% TFA water) was collected in 1.5 mL Eppendorf tubes after passing through the column. All of the fractions were dried, and the dried fractions of the same ACN concentration were reconstituted into 1 tube in 1 mL of 0.05% TFA water for further purification via HPLC or reconstituted in 1 mL of phosphate buffered saline for use in cell treatments.

A Waters 2695 separations module with a photodiode array detector was used to purify the 10% ACN extract. An XterraRP C18 column (4.6 X 150 mm) was used for the separation with 0.05% TFA water as the mobile phase. Each HPLC run was a 20 minute gradient ranging from 0 to 2.5% ACN. The injection volume was 100 µL, and the flow rate was 0.5 mL/minute. HPLC fractionation of the 10% ACN extract yielded three major UV absorbing peaks eluted at 3.5, 3.9, and 12.1 minutes (Figure 1). Collection and HPLC re-purification of the 3.5 minute fraction yielded a symmetrical peak displaying a maximum absorbance at 198.3 nm (Figure 2).

The three peaks were evaluated to determine whether or not they obtained material having the ability to alter the expression levels of polypeptides involved in mitochondria activity and function. Briefly, 5 x 10⁵ neuroblastoma cells obtained from American Type Culture Collection (ATCC) were plated into each well of 6-well plates with 2 mL of RPMI media and incubated for 4 hours in the presence or absence of different aliquots of the HPLC purified material. Following the incubation, total RNA was isolated and purified using the RNeasy mini kit (Qiagen, Valencia, CA). In particular, pelleted cells were resuspended in 600 µL of RLT lysis buffer (Qiagen) and homogenized by passing the lysate 20 times through a 1 mL pipette tip. The samples were then processed according to the manufacturer's instructions (Qiagen, Valencia, Ca). In the final step, the RNA was eluted with 40 µL of RNase-free water by centrifugation for 1 minute at 13,000g. The RNA was analyzed on a model 2100 bioanalyzer (Agilent, Santa Clara, CA) using a total RNA nanochip according to the manufacturer's protocol. Afterwards, 2 µg of total RNA was reverse transcribed using Superscript III reverse transcriptase and random primers.

DNA microarray analyses also were performed using a system provided by Agilent. Arrays included four arrays per chip (Agilent 4X44K chips). Total RNA was reverse transcribed (400 ng) using T7 primers and labeled and transcribed using Cyanine-3 dye. Each array was hybridized with at least 1.65 µg of labeled cRNA at 65°C for 18 hours. Arrays were scanned using an Agilent array scanner. A 10% or greater change in gene expression was capable of being determined using both microarray platforms.

Incubation of cultured cells with the HPLC purified fraction eluted at 3.5 minutes produced changes in the expression of mitochondrial and cellular metabolic genes (Table 1). The extracted potato material that eluted at 3.5 minutes is referred to herein as potosaccharide material or a potosaccharidc preparation since it was determined to a polysaccharide as indicated below. The 3.5 minute fraction (a potosaccharide preparation) was the only fraction of the three determined to possess significant biological activity when tested using real time PCR for TFAM, NFKB, and HMGCR expression.

**Table 1. Gene expression changes in HTB-11 cells as determined by microarray following a four-hour incubation with a potosaccharide preparation.**

| Gene symbol | Gene name | % change |
|---|---|---|
| TFAM | transcription factor A, mitochondrial | +15 |
| FOX01A | forkhead box O1 | -28 |
| NFKB1 | nuclear factor of kappa light polypeptide gene enhancer in B-cells 1 | -14 |
| ATP5A1 | ATP synthase, H+ transporting, mitochondrial F1 complex, alpha subunit 1 | +30 |
| PDHA1 | pyruvate dehydrogenase (lipoamide) alpha 1 | +8 |
| PDHA2 | pyruvate dehydrogenase (lipoamide) alpha 2 | +41 |
| PDK2 | pyruvate dehydrogenase kinase, isozyme 2 | -24 |
| PDK4 | pyruvate dehydrogenase kinase, isozyme 4 | -41 |
| HMGCR | 3-hydroxy-3-methylglutaryl-CoA reductase | -18 |
| THOP1 | thimet oligopeptidase 1 | +23 |

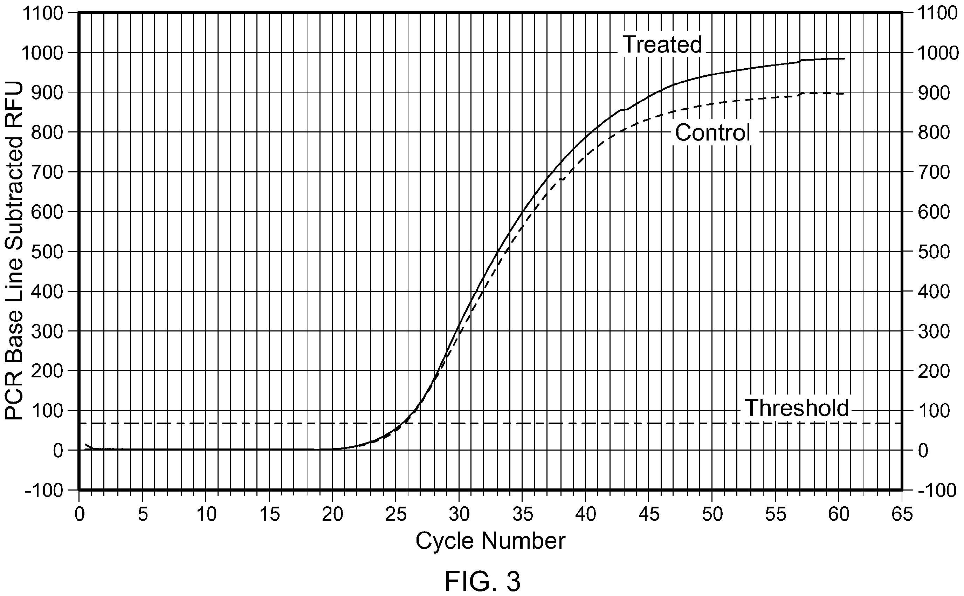

Real-time PCR was performed in triplicate with TFAM, HMGCR, and NFKB1 detector sets. Beta-actin or GAPDH was used as a reference gene. The real-time PCR master mix included 25 µL 2x universal master mix, 2.5 µL 20x detector set (with the primer and probe), and 21.5 µL of water. PCR was performed in an Applied Biosystems 7500 sequence detection system. The thermocycler conditions included denaturation at 95°C for 15 seconds and annealing/extension at 60°C for 60 seconds. Forty cycles of PCR were preceded by 95°C for 10 minutes. Reactions were performed in triplicate. The relative quantities of TFAM were found using the formula 2^{-ΔΔt} using the Applied Biosystems 7500 software. Validation of some of the microarray results by real time PCR used TFAM, HMGCR, and NFKB1 as candidate genes. A representative real time PCR amplification plot demonstrated that TFAM mRNA was present and was differentially expressed (Figure 3). The potosaccharide preparation had a profound effect on TFAM expression and was able to upregulate it by 57% (Table 2). Both HMGCR and NFKB1 gene expression were reduced by approximately 20%, consistent with and validating the DNA microarray data (Table 2).

**Table 2. Validation of gene expression changes by real time PCR. HTB-11 cells treated for 4 hours with a potosaccharide preparation.**

| Gene Symbol | % change |
|---|---|
| TFAM | +57 ± 9 |
| NFKB1 | -20 ± 5 |
| HMGCR | -19 ± 4 |

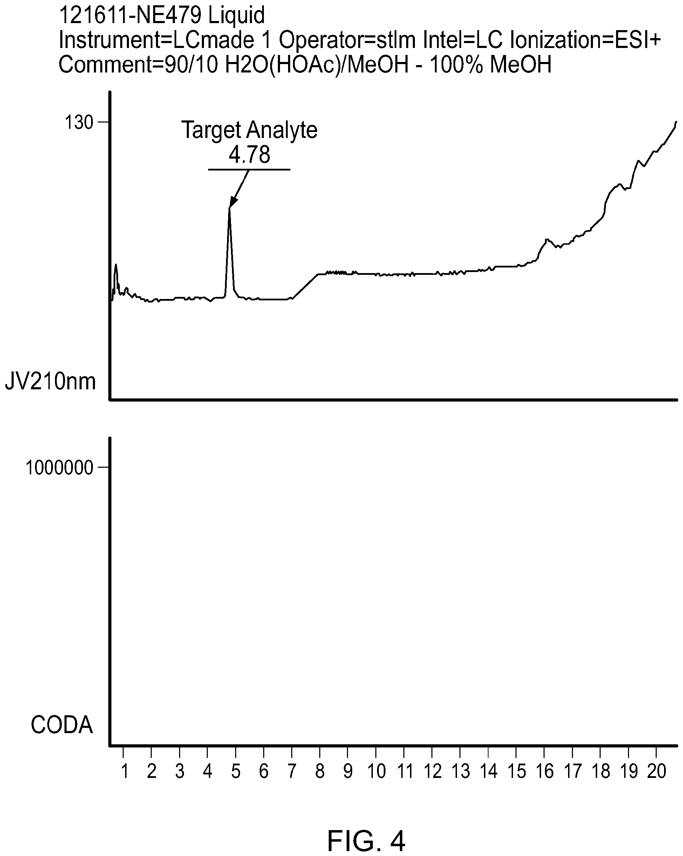

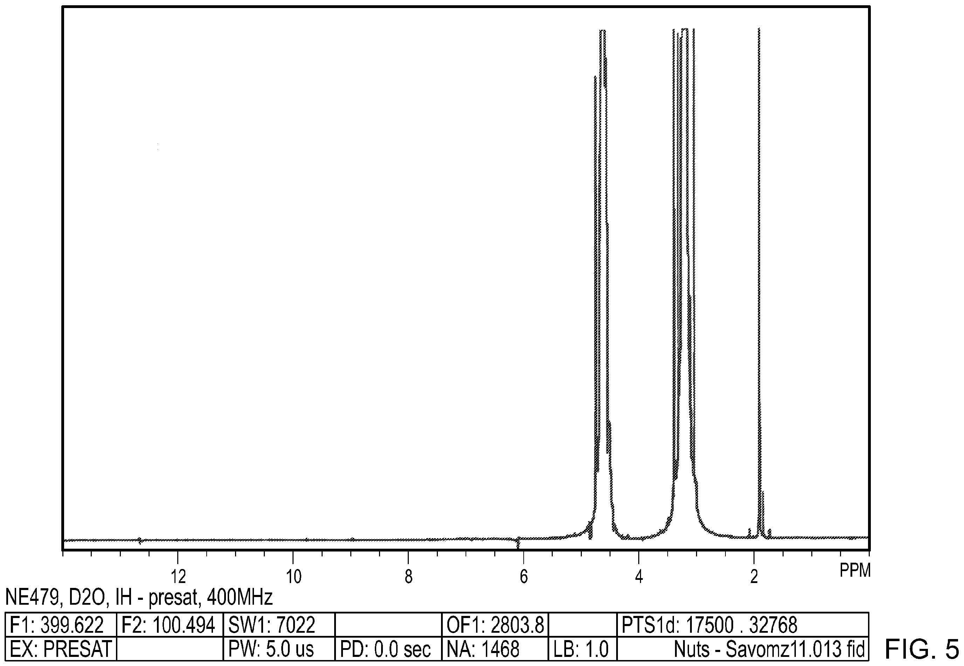

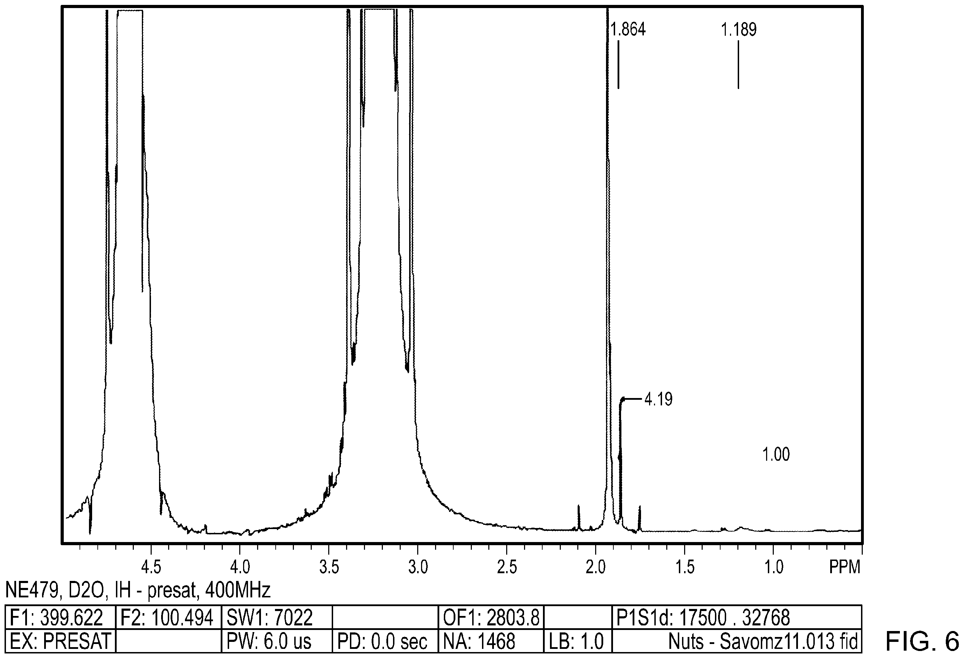

Further chemical characterization of the symmetrical 3.5 minute HPLC peak material was performed. Pooled 3.5 minute HPLC fractions were dried and reconstituted in 1 mL TFA water and subjected to tandem LC/MS/MS (Figure 4) and NMR chemical analyses (Figures 5 and 6). For the NMR analysis, ¹H-NMR was run on the sample using deuterium oxide (D₂O) as a solvent to further analyze the sample. The water peak at 4.65 PPM was solvent-suppressed, and the spectrum was acquired for several hours. Acetamide was detected at 3.2 PPM, along with acetonitrile at 1.9 PPM. Minor peaks were detected at 1.05 PPM, 1.17 PPM (broad peak), 1.189 PPM, and 1.864 PPM. One characteristic of polymeric materials in a proton NMR was the broadening of peaks such as the shift at 1.17 PPM. These shifts on the NMR could represent the peak at 4.8 PPM and suggested a polar, water-soluble polymer such as a polysaccharide. Taken together, these results confirmed the presence of high molecular weight polysaccharide material contained in HPLC purified fractions eluting at 3.5 minutes.

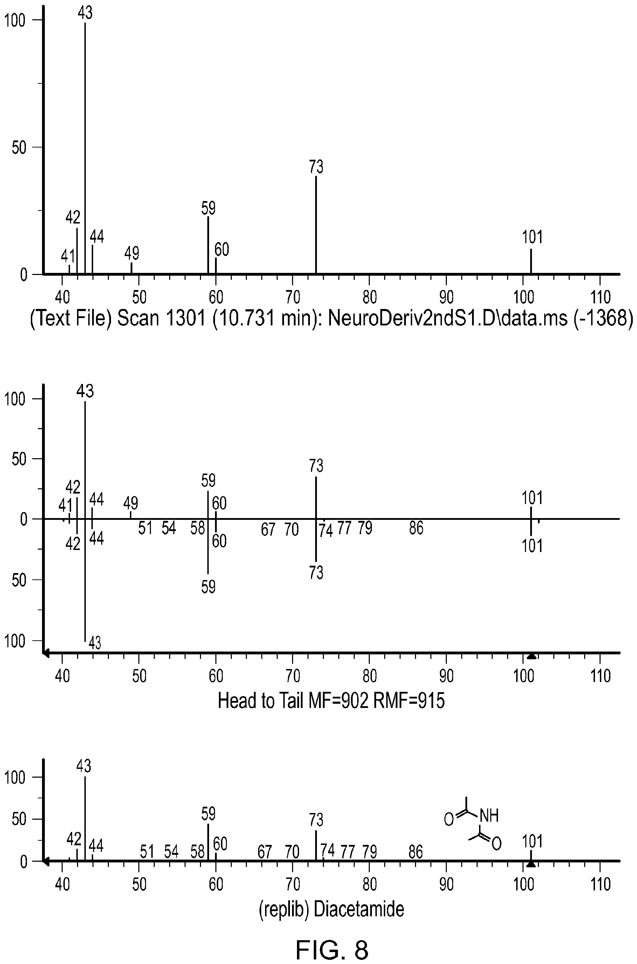

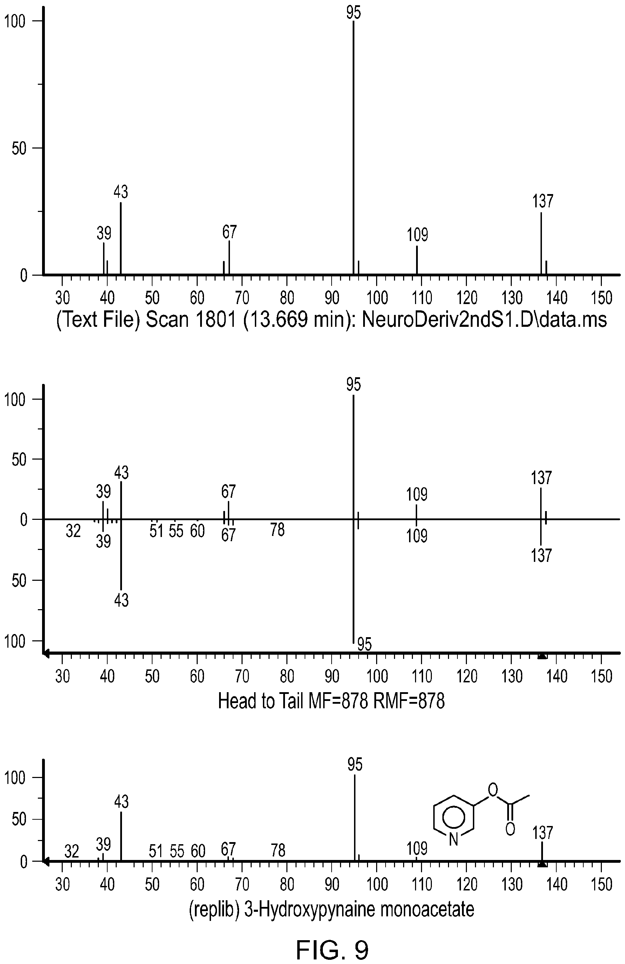

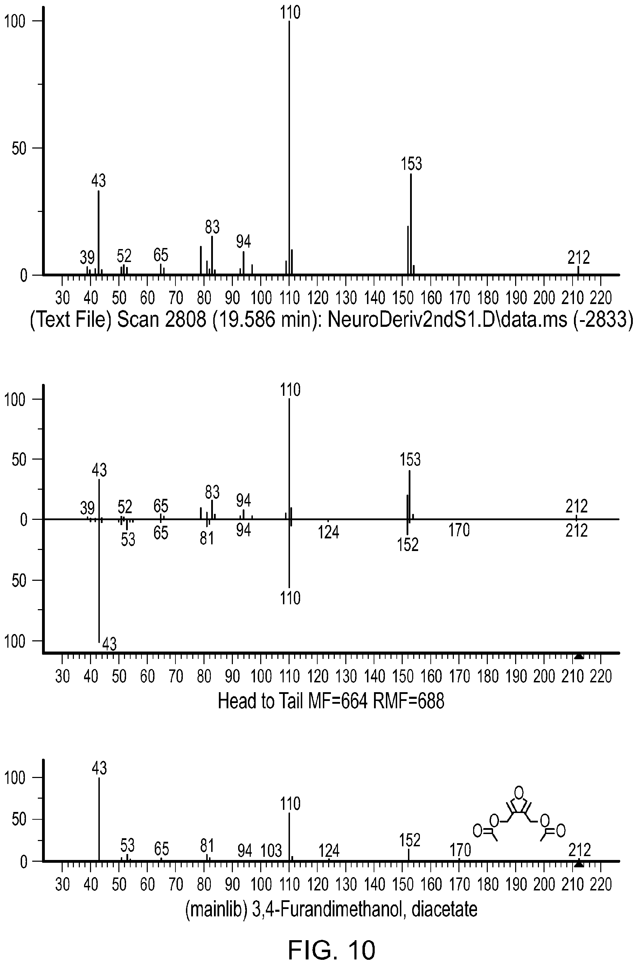

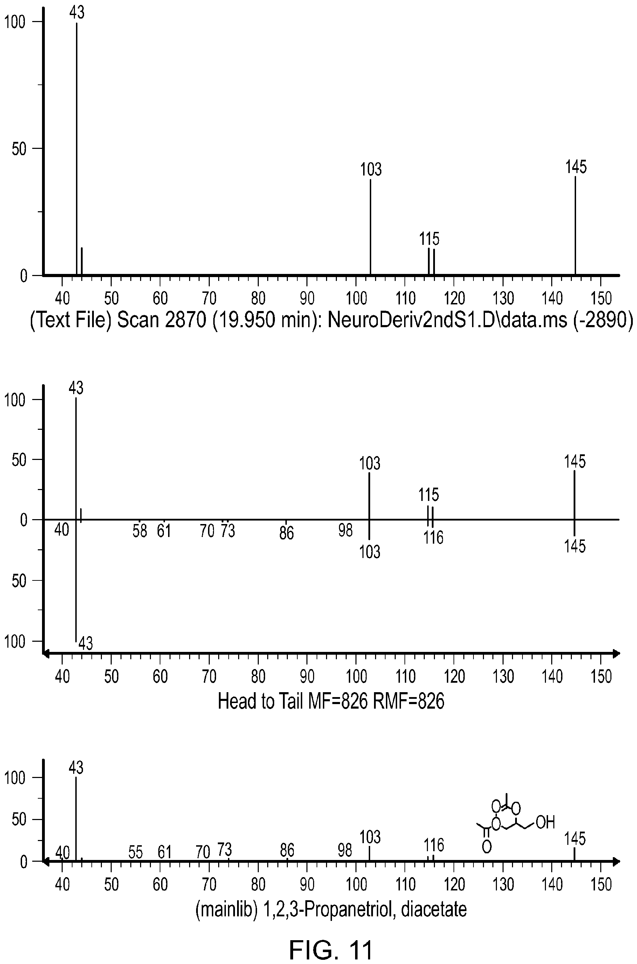

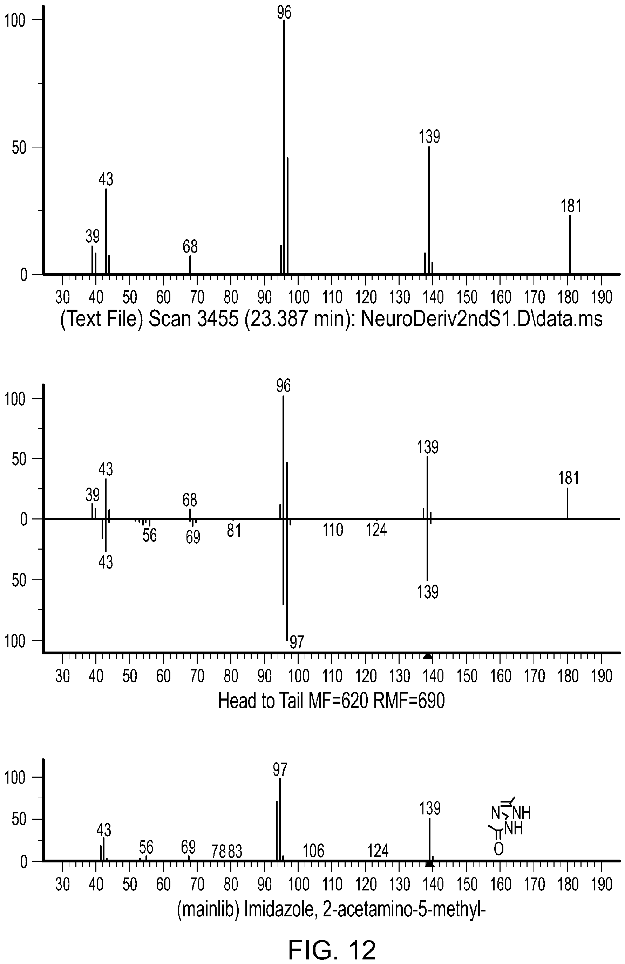

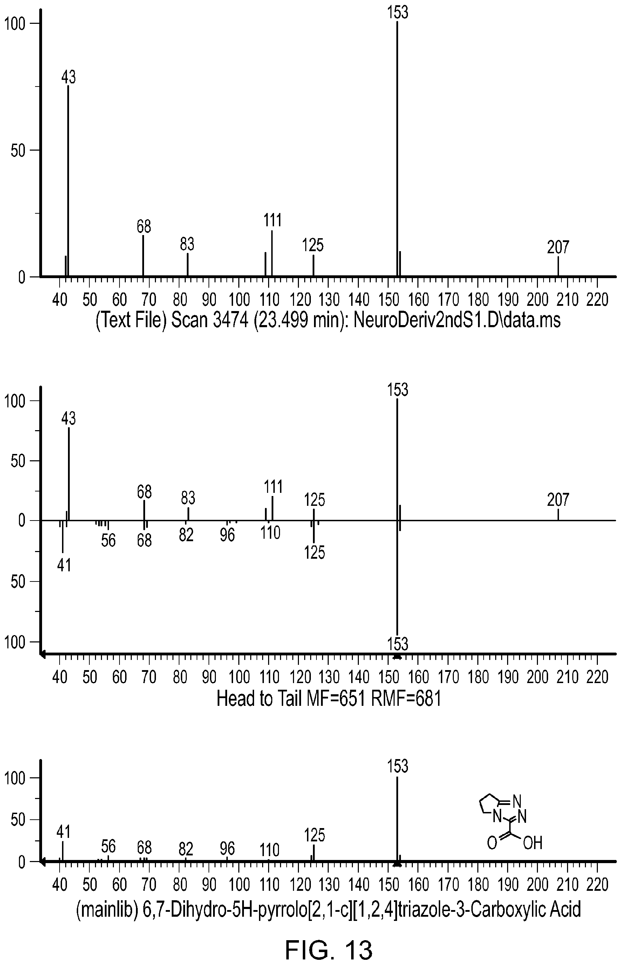

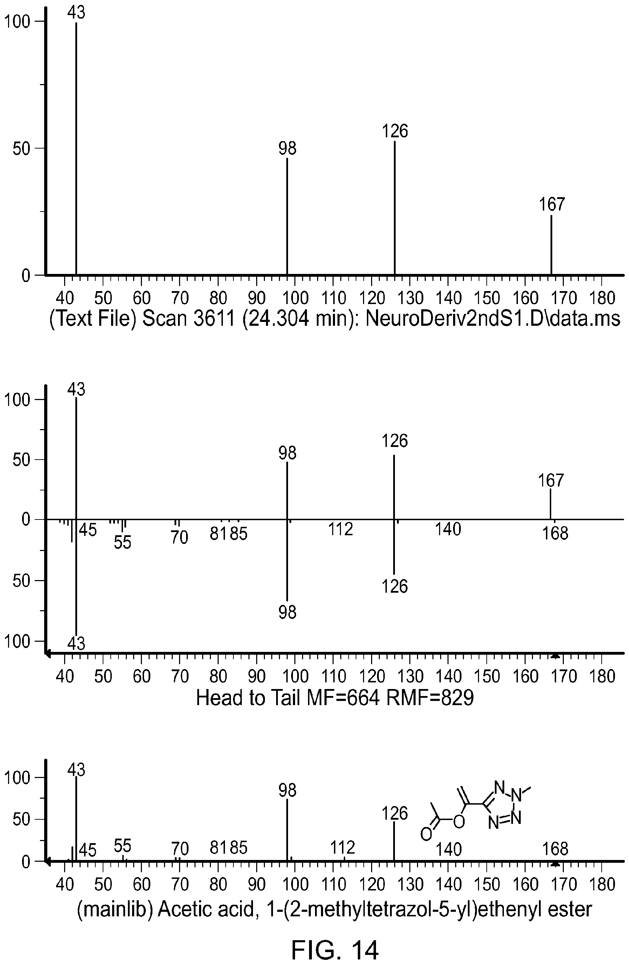

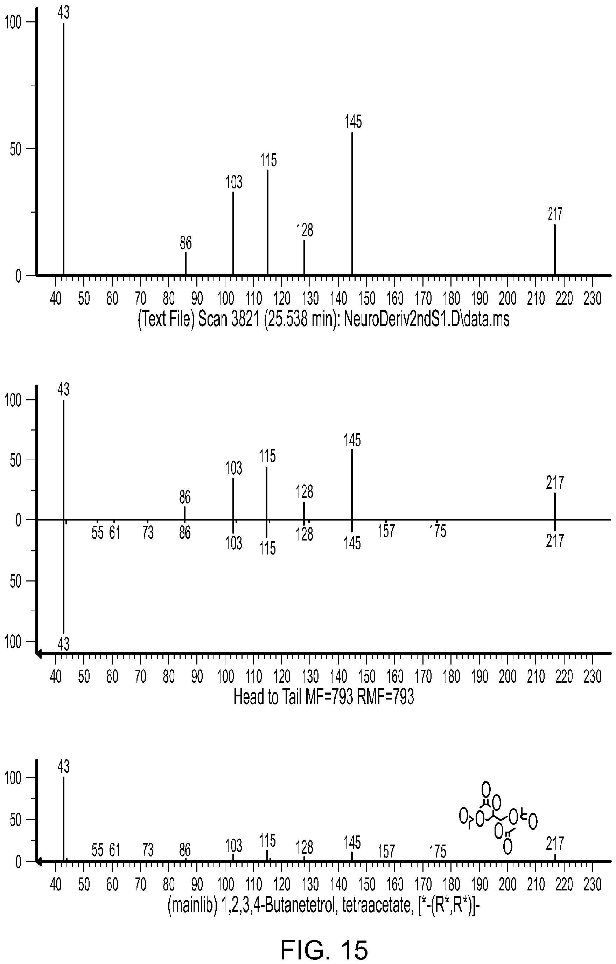

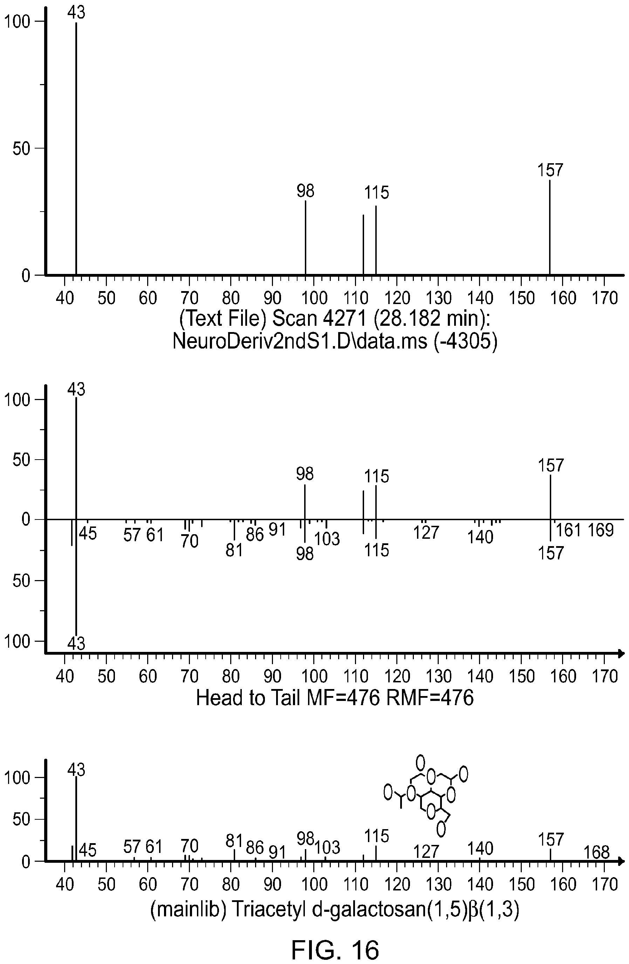

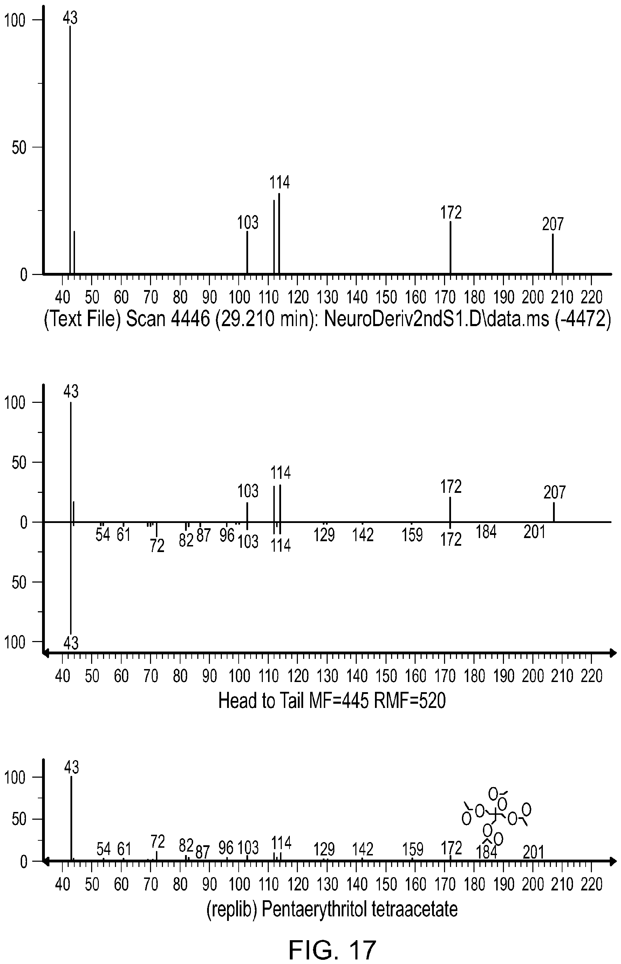

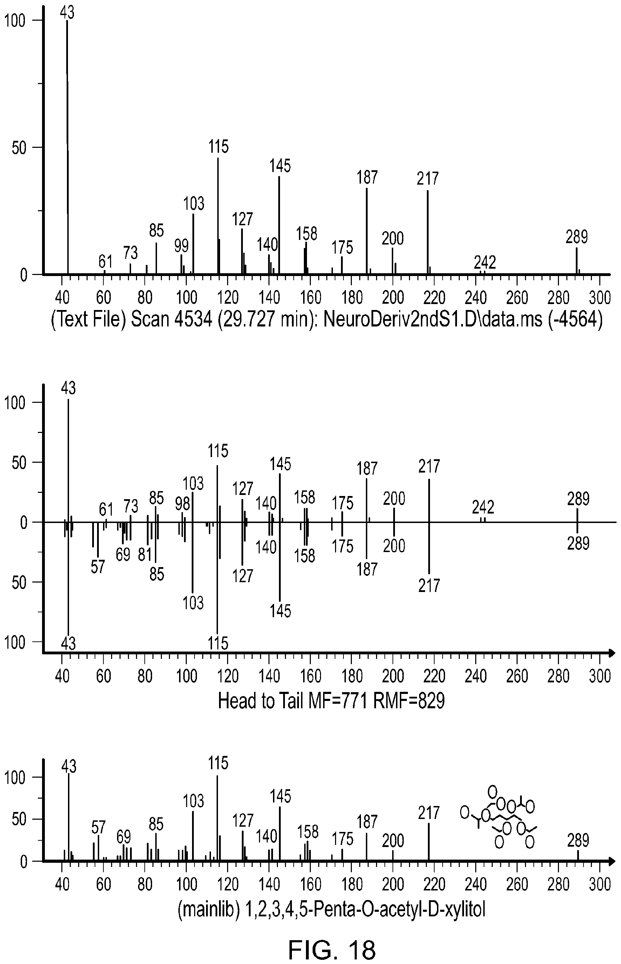

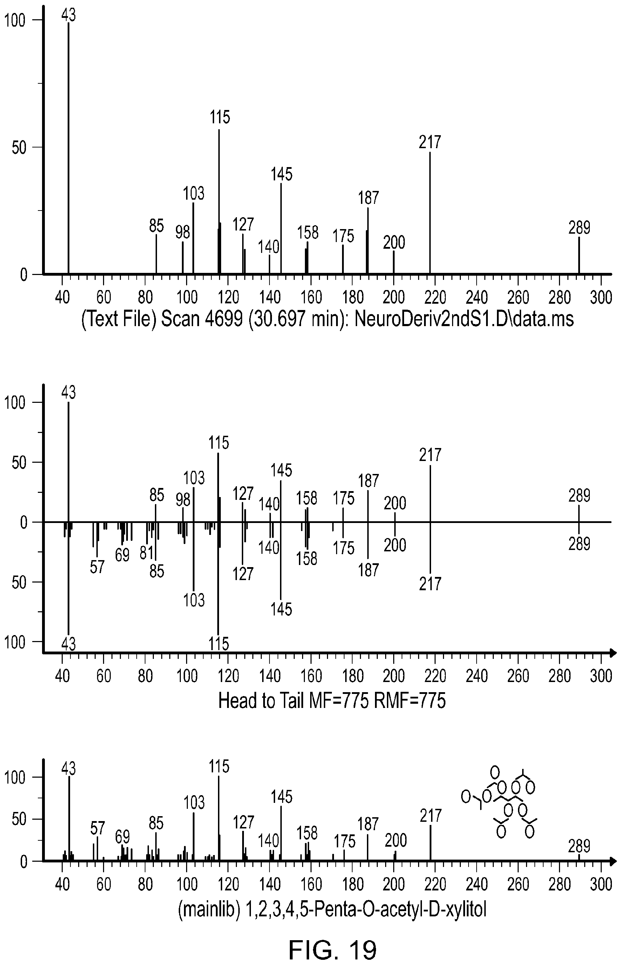

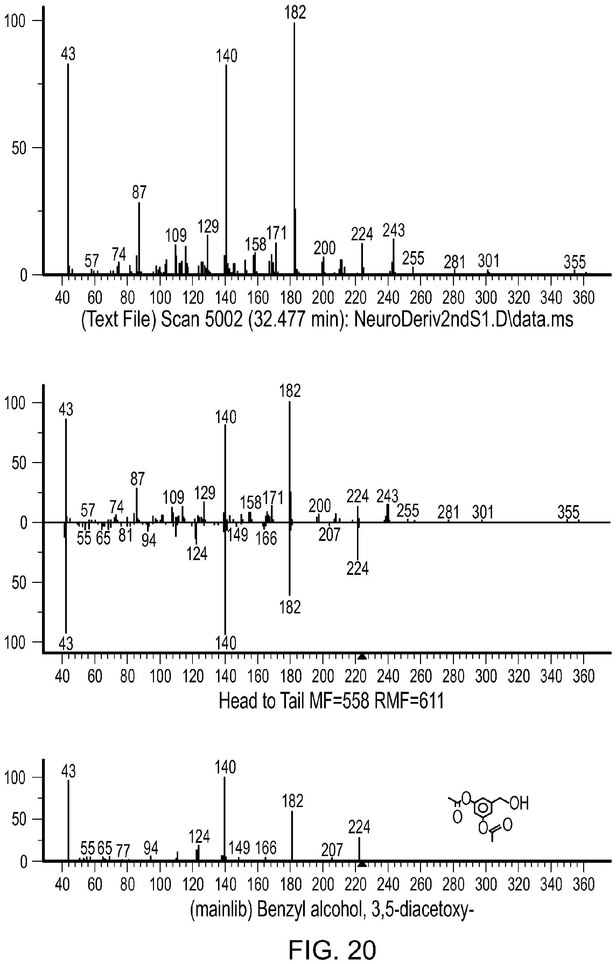

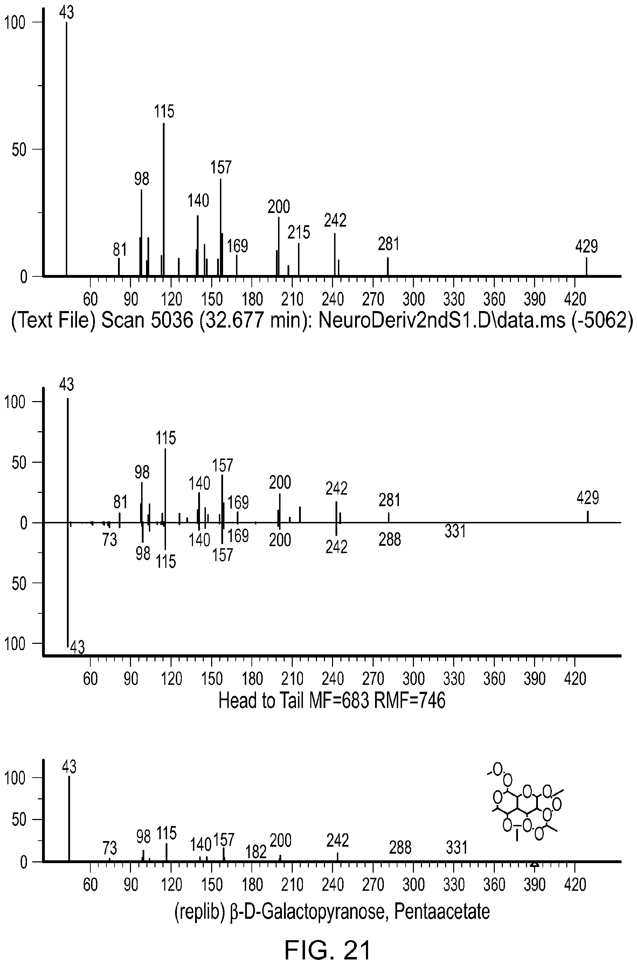

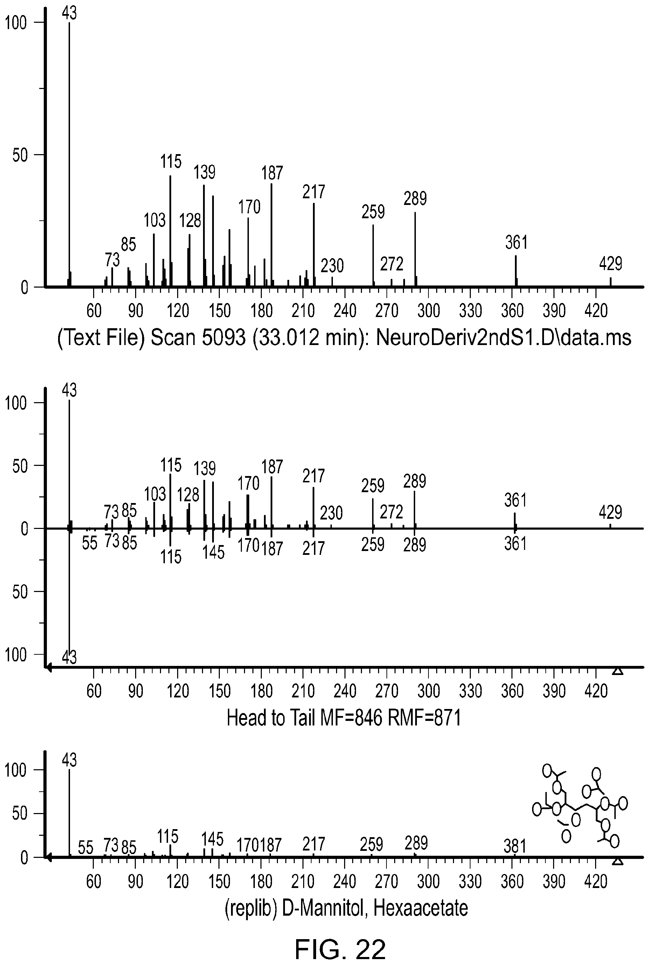

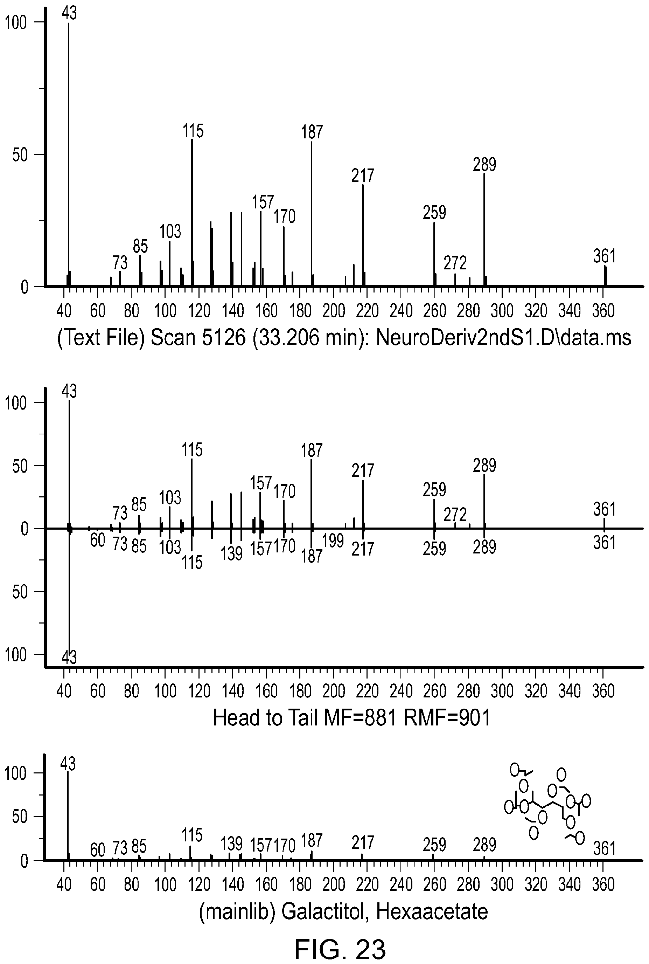

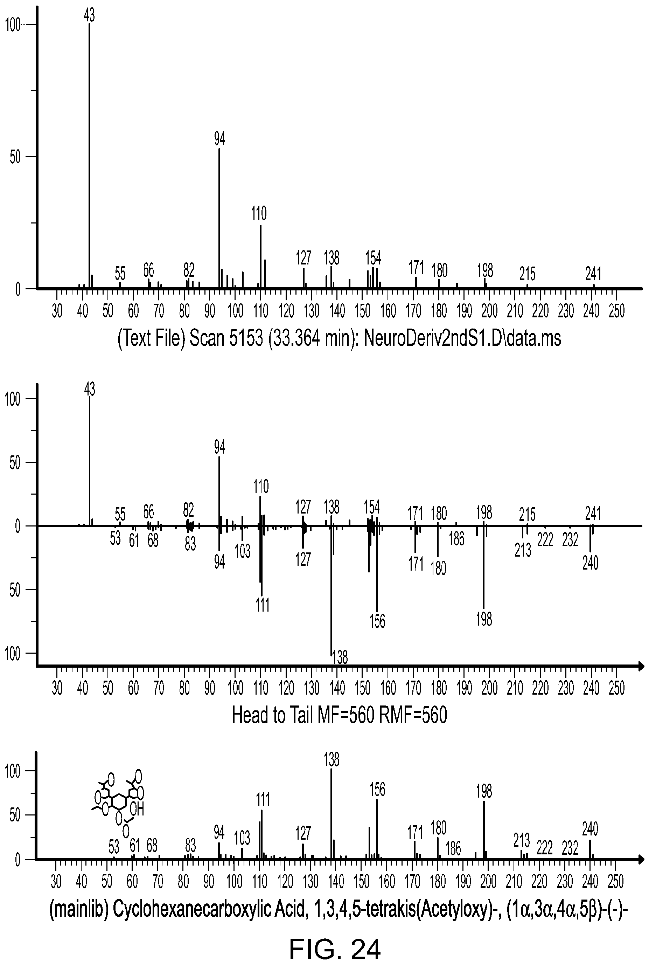

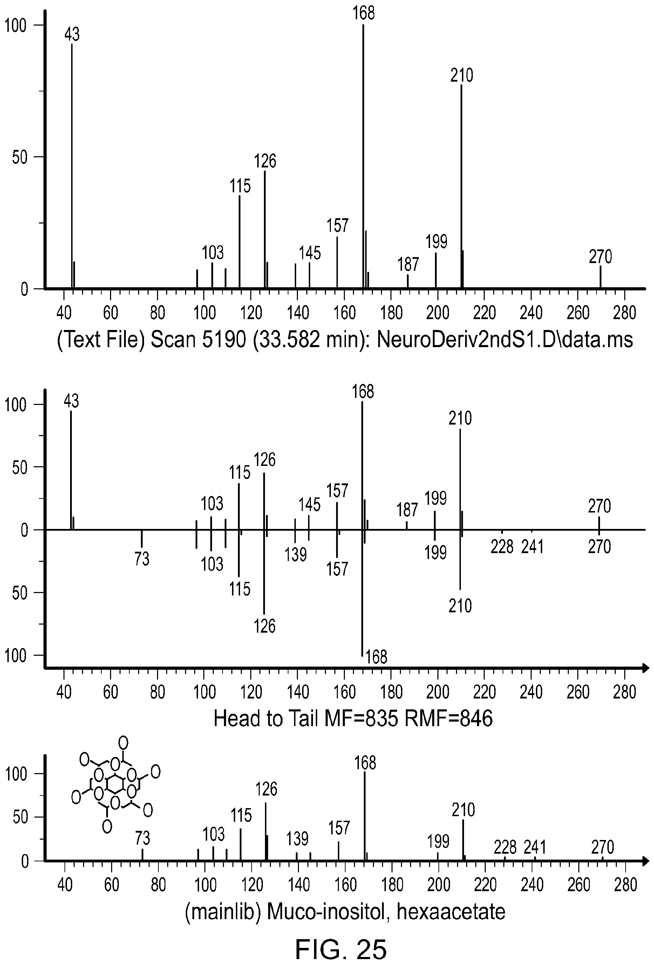

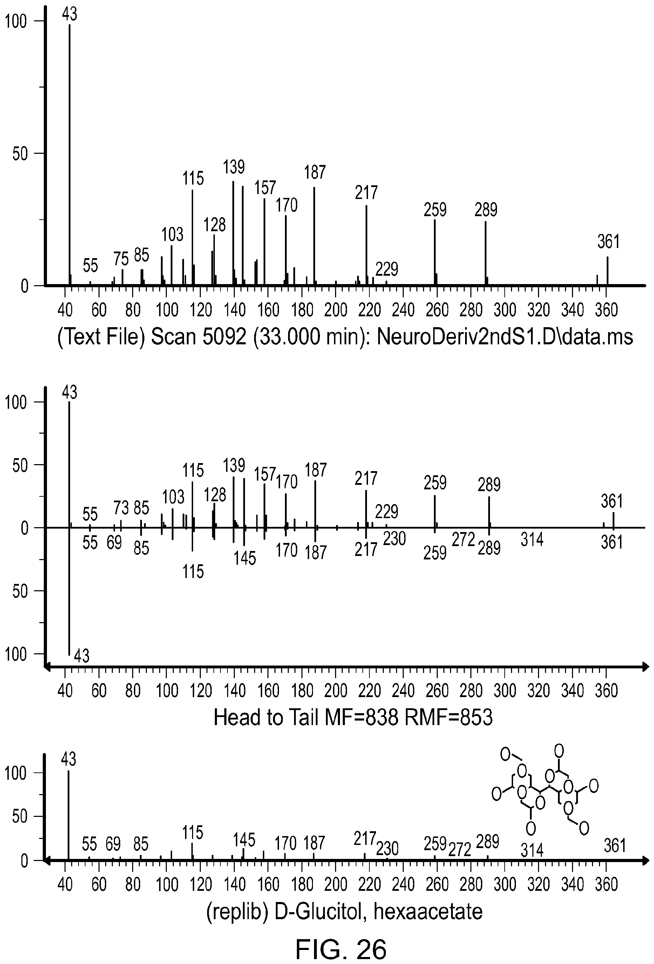

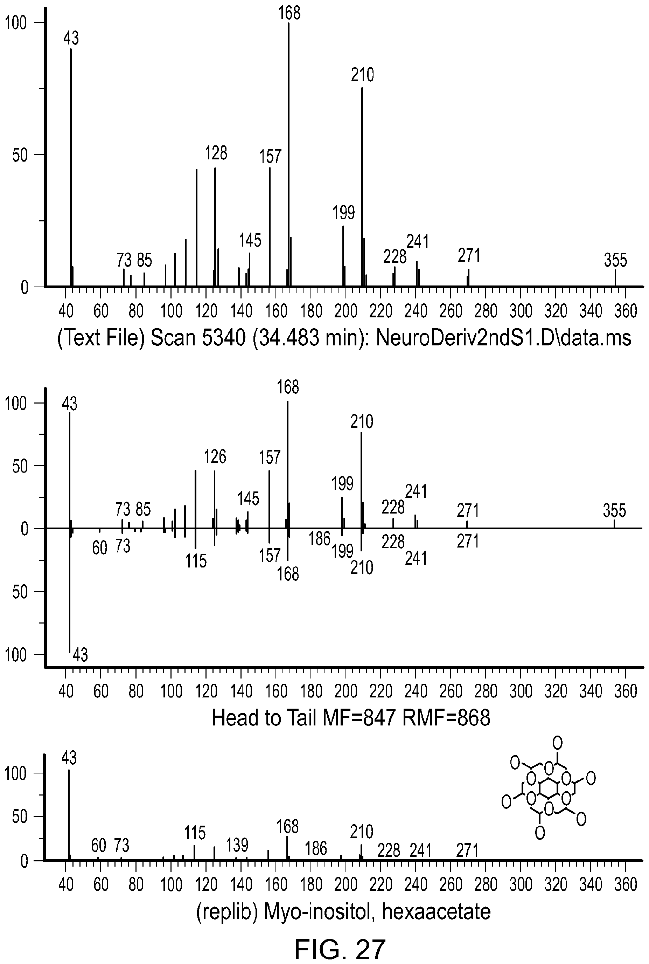

Further analysis confirmed that the HPLC purified fraction eluting at 3.5 minutes contains polysaccharide material (e.g., highly substituted complex xyloglucan material). To make the polysaccharide material analyzable by gas chromatography/mass spectroscopy (GC/MS), it was converted into its derivatized carbohydrate fragments. Briefly, the sample was concentrated to a dry residue that was hydrolyzed using trifluoroacetic acid. This was then reduced using sodium borohydride, and after borate removal, the end product was acylated using acetic anhydride and pyridine. The end products of the reaction were injected directly on GC/MS to identify any acylated carbohydrates. Based on the end analysis, a larger carbohydrate existed in the sample. The total ion chromatogram (TIC) is shown below in Figure 7 with appropriate peak labels below in Table 3. The major components identified are indicated in bold (peaks 3, 12, 14, and 21). The corresponding fragmentation for each compound is provided in Figures 8-27. For each fragmentation, the peak fragmentation pattern is on the top, the compound library fragmentation match is on the bottom, and an overlay of the two is in the center. Finally, unlabeled peaks were either column bleed or did not have a sufficient match to the compound library.

**Table 3: Summary of GC/MS results.**

| **Peak** | **Retention Time (min)** | **Compound Name** | **Structure** |
|---|---|---|---|
| 1 | 10.731 | Diacetamide | 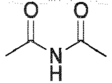 |
| 2 | 13.669 | 3-Acetoxy pyridine | 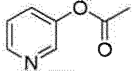 |
| 3 | **19.568** | **3,4-Furan dimethanol, diacetate** | 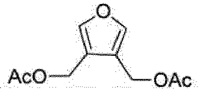 |
| 4 | 19.950 | 1,2,3-propanetriol diacetate | 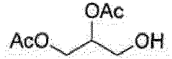 |
| 5 | 23.387 | Imidazole, 2-acetamino-5-methyl | 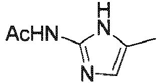 |
| 6 | 23.499 | 6,7-dihydro-5H-pyrrol[2,1,c][1,2,4] triazole-3 -carboxylic acid | 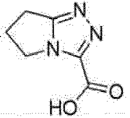 |
| 7 | 24.304 | Acetic acid, 1-(2-methyltetrazol-5-yl) ethenyl ester | 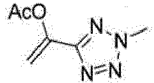 |
| 8 | 25.538 | 1,2,3,4-butanetriol, tetraacetate | 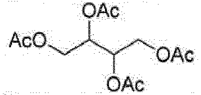 |
| 9 | 27.412 | (1,5)β(1,3)triacetyl D-galactosan (stereoisomer 1) | 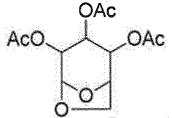 |
| 10 | 28.188 | (1,5)β(1,3)triacetyl D-galactosan (stereoisomer 2) | 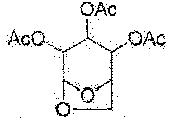 |
| 11 | 29.210 | Pentaerythritol tetraacetate | 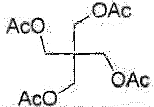 |
| 12 | **29.727** | **1,2,3,4,5-penta-o-acetyl-D-xylitol (isomer 1)** | 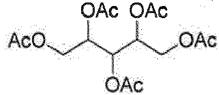 |
| 13 | 30.697 | 1,2,345-penta-o-acetyl-D-xylitol (isomer 2) | 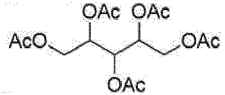 |
| **14** | **32.477** | **3,5-diacetoxy-benzyl alcohol** | 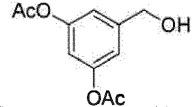 |
| 15 | 32.677 | β-D-glucopyranose, pentaacetate | 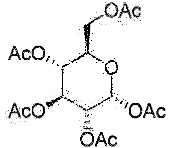 |
| 16 | 33.012 | D-mannitol hexaacetate | 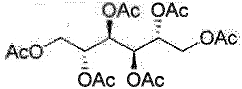 |
| 17 | 33.106 | β-D-galactopyranose, pentaacetate | 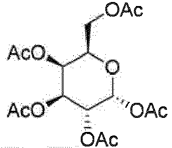 |
| 18 | 33.206 | Galacticol, hexaacetate | 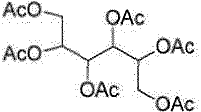 |
| 19 | 33.364 | Cyclohexane carboxylic acid, 1,2,45-tetrakis(acetoxy), (1α,3α,4α,5β)-(-) | 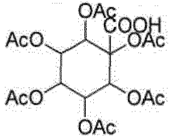 |
| 20 | 33.582 | Muco-inositol, hexaacetate | 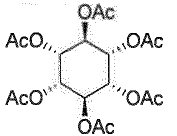 |
| **21** | **33.006** | **D-glucitol-hexaacetate** | 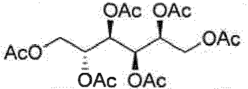 |
| 22 | 34.463 | Myo-inositol, hexaacetate | 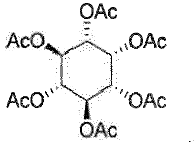 |

These results demonstrate the presence of sugar monomers that serve as building blocks for a larger carbohydrate. It appeared from these multiple lines of analysis that the potosaccharide preparation is a highly substituted complex xyloglucan.

### Example 2 - Sweet potatoes and multiple varieties of potatoes exhibit the presence of potosaccharide material

Six grams of potato material from multiple varieties of *Solanum tuberosum* (Organic yellow, Purple, Idaho Russet, and Yukon Gold) and six grams of material from sweet potatoes (*Ipomoea batatas*) were extracted in 20 mL of water. 10 mL of that water was then loaded onto a sep-pak cartridge, and the cartridge was then eluted with 10 mL of 10% ACN. The ACN was then dried, and the residue was dissolved in 1 mL of water. A 100 µL injection of this water was assessed using HPLC.

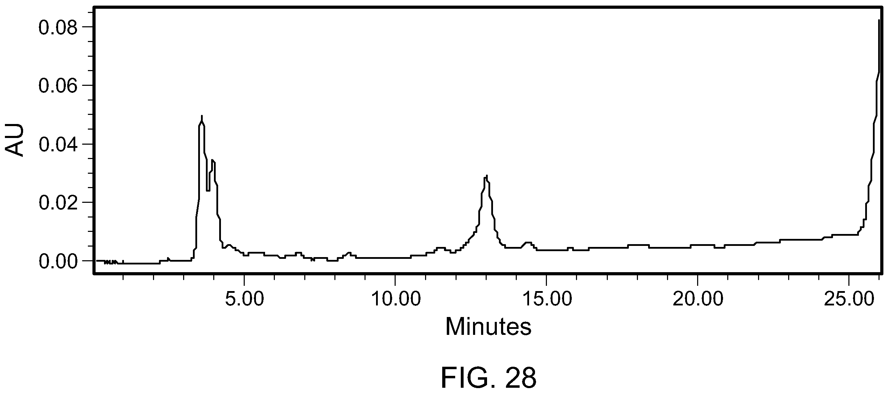

The HPLC chromatograms demonstrated that the amount of the first peak (at 3.5 minutes at 210 nm) was the same for all five types of potatoes tested (Figures 28-32).

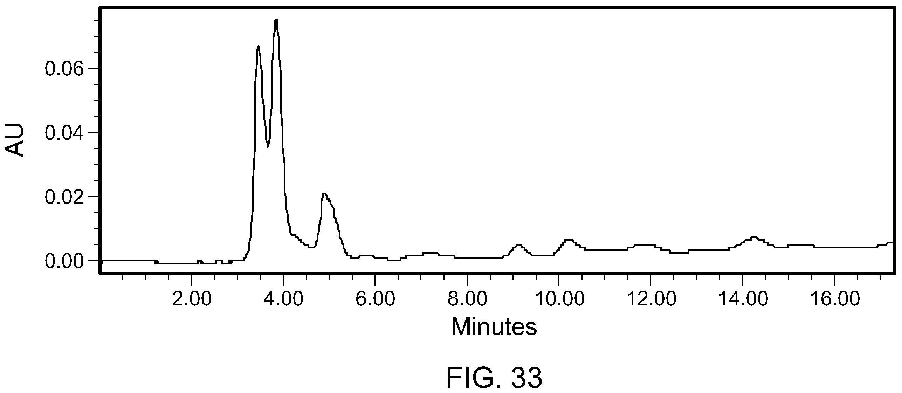

In another experiment, material was extracted from a boiled Purple potato and analyzed. The peak at 3.5 minutes was not reduced in the boiled potato (Figure 33).

The 3.5 minute peak from two pooled fraction collections from Idaho Russet potatoes was collected, dried, and reconstituted in 100 µL of water. The material was then injected into the HPLC yielding a single peak at 3.5 minutes (Figure 34). Taken together, these results demonstrate that potatoes within the *Solanum tuberosum* and *Ipomoea batatas* species contain potosaccharide material.

### Example 3 - Highly substituted complex xyloglucan from potato material alters expression of polypeptides in human omental apidocytes obtained from diabetic patients

Human omental apidocytes obtained from normal and diabetic patients were purchased from Zen-Bio, Inc (Research Triangle Park, NC). The cells were either untreated or treated with 62.5 µg/mL of the 3.5 minute peak from purple potatoes for four hours. After the four hour incubations, the cells were harvested, and a microarray analysis was performed to measure changes in gene expression.

Incubation of human omental apidocytes from diabetic patients with the HPLC purified fraction eluted at 3.5 minutes produced changes in the expression of genes involved in obesity and/or diabetes (Table 4). Incubation of human omental apidocytes from normal humans produced minimal changes in the expression of the genes listed in Table 4 (Table 5).

**Table 4. Gene expression changes as determined by microarray following a four-hour incubation of human omental apidocytes from diabetic patients with a potosaccharide preparation.**

| Gene symbol | % change |
|---|---|
| AGPAT1 | -1 |
| OLR1 | -45 |
| BCAT2 | -9 |
| NFKB1 | -6 |
| SH2B1 | -17 |
| LPL | -24 |
| HMGCR | -9 |
| LIPE | +15 |
| PCK2 | +5 |
| MOGAT1 | +52 |
| PPARGC1a | +59 |
| PPARGClb | +44 |
| SOD2 | +18 |
| NR4A1 | +12 |
| ACAT2 | +13 |

**Table 5. Gene expression changes as determined by microarray following a four-hour incubation of human omental apidocytes from normal humans with a potosaccharide preparation.**

| Gene symbol | % change |
|---|---|
| AGPAT1 | None detected |
| OLR1 | -18 |
| BCAT2 | None detected |
| NFKB1 | -56 |
| SH2B1 | -33 |
| LPL | +18 |
| HMGCR | +16 |
| LIPE | +32 |
| PCK2 | +30 |
| MOGAT1 | +22 |
| PPARGC1a | +26 |
| PPARGC1b | +26 |
| SOD2 | +23 |
| NR4A1 | +45 |
| ACAT2 | +17 |

Real-time PCR was performed in triplicate with AGPAT1, OLR1, BCAT2, NR4A1, and ACAT2 detector sets. Beta-actin or GAPDII was used as a reference gene. The real-time PCR master mix included 25 µL 2x universal master mix, 2.5 µL 20x detector set (with the primer and probe), and 21.5 µL of water. PCR was performed in an Applied Biosystems 7500 sequence detection system. The thermocycler conditions included denaturation at 95°C for 15 seconds and annealing/extension at 60°C for 60 seconds. Forty cycles of PCR were preceded by 95°C for 10 minutes. Reactions were performed in triplicate. Validation of some of the microarray results by real time PCR used AGPAT1, OLR1, BCAT2, NR4A1, and ACAT2 as candidate genes. Real time PCR amplification plots demonstrated that AGPAT1, OLR1, BCAT2, NR4A1, and ACAT2 mRNAs were present and were differentially expressed (Table 6).

**Table 6. Validation of gene expression changes by real time PCR. Human omental apidocytes from diabetic patients treated for 4 hours with a potosaccharide preparation.**

| Gene Symbol | % change |
|---|---|
| AGPAT1 | -13 ± 1 |
| OLR1 | -9 ± 1 |
| BCAT2 | -4 ± 1 |
| NR4A1 | +34 ± 3 |
| ACAT2 | +12 ± 2 |

### Example 4 - Highly substituted complex xyloglucan from potato material alters expression of polypeptides in mouse myocytes

Mouse myoblasts were seeded in 2 mL aliquots into two 75 cm² tissue culture flasks. Cells were left to differentiate into myocytes for 4 days in 5% CO₂ at 37 °C.

Myocytes were detached from flask walls using gentle agitation. Suspended cells were transferred to a 15mL conical tube and centrifuged at 500g for 3 minutes. 2 mL aliquots were seeded into 75 cm² tissue culture flasks for both control and diabetic model cells. The mouse cells were obtained from normal mice and from mice treated with low dose alloxan. The diabetic mice had high blood glucose compared to the normal mice. A potosaccharide preparation (62.5 µg/mL of the 3.5 minute peak from purple potatoes) was added to one control and one diabetic flask, and the cells were incubated for 24 hours.

After the 24 hour incubation, the cells were harvested, and a microarray analysis was performed to measure changes in gene expression. In addition, images were taken of the cells after treatment using a Nikon EclipseTE300 (Morell) inverted microscope coupled with an Optronics digital cameraware at 20x. The images were analyzed on ImageJ software for cell mortality and fiber size. Cell mortality was calculated using a ratio of the number of inactive cells to the number of active cells. Fiber size was calculated using a polygonal lasso tracer and measured in pixel area.

Incubation of mouse myocytes from the diabetic model with the HPLC purified fraction eluted at 3.5 minutes produced changes in the expression of genes involved in obesity and/or diabetes (Table 7). Incubation of mouse myocytes from normal mice produced minimal changes in the expression of the genes listed in Table 7 (Table 8).

**Table 7. Gene expression changes as determined by microarray following a 24-hour incubation of mouse myocytes from the diabetic model with a potosaccharide preparation.**

| Gene symbol | % change |
|---|---|
| NFKB1 | -46 |
| SH2B1 | -35 |
| LPL | -16 |
| HMGCR | +25 |
| LIPE | -46 |
| PCK2 | none |
| SOD2 | +74 |
| NR4A1 | -33 |
| ACAT2 | none |
| PTEN | -22 |
| CASP8 | not detected |

**Table 8. Gene expression changes as determined by microarray following a 24-hour incubation of mouse myocytes from normal mice with a potosaccharide preparation.**

| Gene symbol | % change |
|---|---|
| NFKB1 | 37 |
| SH2B1 | 202 |
| LPL | 139 |
| HMGCR | 105 |
| LIPE | 147 |
| PCK2 | 118 |
| SOD2 | None detected |
| NR4A1 | 200 |
| ACAT2 | 75 |
| PTEN | 96 |
| CASP8 | 104 |

Real-time PCR was performed in triplicate with PTEN and CASP8 detector sets. Beta-actin or GAPDH was used as a reference gene. The real-time PCR master mix included 25 µL 2x universal master mix, 2.5 µL 20x detector set (with the primer and probe), and 21.5 µL of water. PCR was performed in an Applied Biosystems 7500 sequence detection system. The thermocycler conditions included denaturation at 95°C for 15 seconds and annealing/extension at 60°C for 60 seconds. Forty cycles of PCR were preceded by 95°C for 10 minutes. Reactions were performed in triplicate. Validation of some of the microarray results by real time PCR used PTEN and CASP8 as candidate genes. Real time PCR amplification plots demonstrated that PTEN and CASP8 mRNAs were present and were differentially expressed (Table 9).

**Table 9. Validation of gene expression changes by real time PCR. Mouse myocytes from the diabetic model treated for 24 hours with a potosaccharide preparation.**

| Gene Symbol | % change |
|---|---|
| PTEN | -31 ± 4 |
| CASP8 | -72 ± 8 |

### Example 5 - Analysis of a potosaccharide preparation

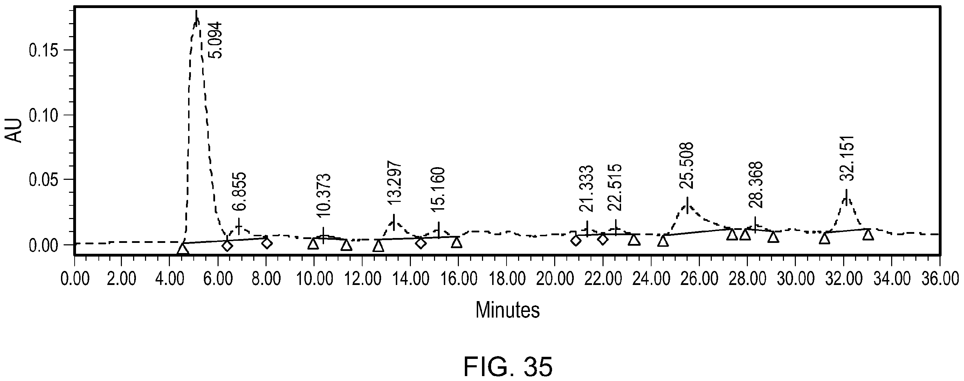

A potosaccharide preparation was purified using HPLC from 3 g of purple potato. The potosaccharide peak was eluted at about 5 minutes (Figure 35). This peak was obtained using a different chromatographic column (10 mm x150 mm) as compared to the column used to obtain the 3.5 minute peak. Since the column was a larger preparative column and the flow rate was 1.5 mL/minute, the elution time of the potosaccharide was 5 minutes.

The obtained peak was collected, dried, and reconstituted in 60 µL of water. The reconstituted potosaccharide material was then added to HTB-11 cells in culture flasks for 4 hours. The post treatment media was collected and added to another flask of HTB-11 cells. Each group of cells was analyzed for gene expression changes. The initially treated cells exhibited the expected changes in mitochondrial gene expression. No changes were detected in the cells exposed to the post treatment media for 4 hours.

In a separate experiment, the post treatment media was extracted using the techniques used to originally purify the potosaccharide. A chromatogram of the extracted post treatment media demonstrated the absence of a peak at 5 minutes.

### Example 6 - Using a potosaccharide preparation to treat obesity Class I-III obese humans are identified based on the criteria of Table 10,

**Table 10. Classification of Overweight and Obesity by BMI, Waist Circumference, and Associated Disease Risks.**

| | | | **Disease Risk* Relative to Normal Weight and Waist Circumference** | |
|---|---|---|---|---|
| | **BMI (kg/m²)** | **Obesity Class** | **Men 102 cm (40 in) or less Women 88 cm (35 in) or less** | **Men > 102 cm (40 in) Women > 88 cm (35 in)** |
| **Underweight** | < 18.5 | | - | - |
| **Normal** | 18.5-24.9 | | - | - |
| **Overweight** | 25.0-29.9 | | Increased | High |
| **Obesity** | 30.0-34.9 | I | High | Very High |
| | 35.0-39.9 | II | Very High | Very High |
| **Extreme Obesity** | 40.0 + | III | Extremely High | Extremely High |

Once identified, a Class I-III obese patient is treated as follows. Potosaccharide is formulated in the presence of alpha lipoic acid or alpha tocopherol or both. Formulated potosaccharide is added to 90% by weight inert binder material and is administered by the oral parenteral route in the form of a tablet, capsule, or liquid, twice daily (bid). Maximal concentrations of potosaccharide are initially administered bid over the course of one month. Positive outcome measures include: (1) significant reduction of BMI, (2) augmentation of serum LDL/HDL ratio, (3) lowering serum triglyceride concentration, (4) lowering systolic and diastolic blood pressure, and (5) lowering fasting blood glucose.

### Example 7 - Using a potosaccharide preparation to treat type II diabetes

Once a type II diabetes patient is identified, the patient is treated as follows. Potosaccharide is formulated in the presence of alpha lipoic acid or alpha tocopherol or both. Formulated potosaccharide is added to 90% by weight inert binder material and is administered by the oral parenteral route in the form of a tablet, capsule, or liquid, twice daily (bid). Maximal concentrations of potosaccharide are initially administered bid over the course of one month. Positive outcome measures include: (1) restoration of normal fasting blood glucose, (2) significant weight loss and lowering of BMI, (3) augmentation of serum LDL/HDL ratio, (4) lowering serum triglyceride concentration, (5) lowering serum concentration of free fatty acids, (6) lowering systolic and diastolic blood pressure, (7) enhancement of insulin sensitivity, and (8) lowering insulin requirement in Type II diabetes patients.

### Example 8 - Using a potosaccharide preparation to treat a polycystic ovary syndrome

Once a polycystic ovary syndrome (POS) patient is identified, the patient is treated as follows. Potosaccharide is formulated in the presence of alpha lipoic acid or alpha tocopherol or both. Formulated potosaccharide is added to 90% by weight inert binder material and is administered by the oral parenteral route in the form of a tablet, capsule, or liquid, twice daily (bid). Maximal concentrations of potosaccharide are initially administered bid over the course of one month. Positive outcome measures include: (1) restoration of normal reproductive function, (2) restoration of normal ovarian follicle maturation, (3) restoration of normal fasting blood glucose levels, (4) significant weight loss and lowering of BMI, (5) augmentation of serum LDL/HDL ratio, (6) lowering serum triglyceride concentration, (7) lowering serum concentration of free fatty acids, (8) lowering systolic and diastolic blood pressure, (9) enhancement of insulin sensitivity, and (10) lowering insulin requirement in comorbid POS patients with type II diabetes.

## Claims

1. A nutritional supplement composition comprising a potato polysaccharide preparation obtained from raw potatos, wherein the potato polysaccharide preparation comprises at least 3,4-Furandimethanol,diacetate, 1,2,3,4,5-penta-o-acetyl-D-xylitol, 3,5-diacetoxy-benzylalchohol and D-glucitol-hexaacetate, wherein at least 80 percent of said potato polysaccharide preparation is potato polysaccharide.

2. The composition of claim 1, wherein said composition comprises:
a) between 1 mg and 100 mg of said potato polysaccharide preparation;
b) between 6 mg and 20 mg of said potato polysaccharide preparation;
c) between 1 mg and 100 mg of the potato polysaccharide component of said potato polysaccharide preparation; or
d) between 6 mg and 20 mg of the potato polysaccharide component of said potato polysaccharide preparation.

3. The composition of claim 1, wherein said composition is in the form of a tablet.

4. The composition of claim 1, wherein said composition comprises alpha lipoic acid or alpha tocopherol.

5. The composition of claim 1, wherein at least a) 90 percent; or c) 95 percent of said potato polysaccharide preparation is potato polysaccharide.

6. The polysaccharide preparation of claim 1 obtained from raw potatoes for use in:
a) increasing polypeptide expression in cells, wherein said cells are to be contacted with said potato polysaccharide preparation under conditions wherein expression of one or more of the polypeptides selected from the group consisting of a TFAM polypeptide, an ATP5A1 polypeptide, a PDHA1 polypeptide, a PDHA2 polypeptide, and a THOP1 polypeptide is increased;
b) reducing polypeptide expression in cells, wherein said cells are to be contacted with said potato polysaccharide preparation under conditions wherein expression of one or more of the polypeptides selected from the group consisting of a FOXOIA polypeptide, a NFKB1 polypeptide, a PDK2 polypeptide, a PDK4 polypeptide, and a HMGCR polypeptide is reduced; or
c) treating obesity, diabetes, and/or polycystic ovary syndrome in a mammal, wherein
(i) said mammal is to be identified having said obesity, diabetes, and/or polycystic ovary syndrome, and
(ii) said potato polysaccharide preparation is to be administering to said mammal.

## Patentansprüche

1. Nahrungsergänzungszusammensetzung, umfassend eine Kartoffelpolysaccharidzubereitung, die aus rohen Kartoffeln gewonnen wird, wobei die Kartoffelpolysaccharidzubereitung mindestens 3,4-Furandimethanol-Diacetat, 1,2,3,4,5-Penta-o-acetyl-D-xylit, 3,5-Diacetoxybenzylalkohohol und D-Glucitol-Hexaacetat umfasst, wobei es sich bei mindestens 80 Prozent der Kartoffelpolysaccharidzubereitung um Kartoffelpolysaccharid handelt.

2. Zusammensetzung nach Anspruch 1, wobei die Zusammensetzung umfasst:
a) zu zwischen 1 mg und 100 mg die Kartoffelpolysaccharidzubereitung;
b) zu zwischen 6 mg und 20 mg die Kartoffelpolysaccharidzubereitung;
c) zu zwischen 1 mg und 100 mg die Kartoffelpolysaccharidkomponente der Kartoffelpolysaccharidzubereitung; oder
d) zu zwischen 6 mg und 20 mg die Kartoffelpolysaccharidkomponente der Kartoffelpolysaccharidzubereitung.

3. Zusammensetzung nach Anspruch 1, wobei die Zusammensetzung in Form einer Tablette vorliegt.

4. Zusammensetzung nach Anspruch 1, wobei die Zusammensetzung alpha-Liponsäure oder alpha-Tocopherol umfasst.

5. Zusammensetzung nach Anspruch 1, wobei es sich bei mindestens a) 90 Prozent; oder c) 95 Prozent der Kartoffelpolysaccharidzubereitung um Kartoffelpolysaccharid handelt.

6. Polysaccharidzubereitung nach Anspruch 1, die aus rohen Kartoffeln gewonnen wurde, zur Verwendung zur:
a) Erhöhung der Polypeptidexpression in Zellen, wobei die Zellen mit der Kartoffelpolysaccharidzubereitung unter Bedingungen in Kontakt gebracht werden, bei denen die Expression von einem oder mehreren der Polypeptide, die ausgewählt sind aus der Gruppe bestehend aus einem TFAM-Polypeptid, einem ATP5A1-Polypeptid, einem PDHA1-Polypeptid, einem PDHA2-Polypeptid und einem THOP1-Polypeptid, erhöht ist;
b) Verringern der Polypeptidexpression in Zellen, wobei die Zellen mit der Kartoffelpolysaccharidzubereitung unter Bedingungen in Kontakt gebracht werden, bei denen die Expression von einem oder mehreren der Polypeptide, die ausgewählt sind aus der Gruppe bestehend aus einem FOX01A-Polypeptid, einem NFKB1-Polypeptid, einem PDK2-Polypeptid, einem PDK4-Polypeptid und einem HMGCR-Polypeptid, verringert ist; oder
c) Behandlung von Adipositas, Diabetes und/oder polyzystischem Ovarialsyndrom in einem Säuger, wobei
(i) festgestellt wird, dass der Säuger Adipositas, Diabetes und/oder polyzystisches Ovarialsyndrom hat, und
(ii) die Kartoffelpolysaccharidzubereitung an den Säuger verabreicht wird.

## Revendications

1. Composition de complément nutritionnel comprenant une préparation de polysaccharide de pomme de terre obtenue à partir de pommes de terre crues, dans laquelle la préparation de polysaccharide de pomme de terre comprend au moins du diacétate de 3,4-furanediméthanol, du 1,2,3,4,5-penta-O-acétyl-D-xylitol, de l'alcool 3,5-diacétoxybenzylique et de l'hexaacétate de D-glucitol, dans laquelle au moins 80 pour cent de ladite préparation de polysaccharide de pomme de terre est du polysaccharide de pomme de terre.

2. Composition selon la revendication 1, ladite composition comprenant :
a) entre 1 mg et 100 mg de ladite préparation de polysaccharide de pomme de terre ;
b) entre 6 mg et 20 mg de ladite préparation de polysaccharide de pomme de terre ;
c) entre 1 mg et 100 mg du composant polysaccharide de pomme de terre de ladite préparation de polysaccharide de pomme de terre ; ou
d) entre 6 mg et 20 mg du composant polysaccharide de pomme de terre de ladite préparation de polysaccharide de pomme de terre.

3. Composition selon la revendication 1, ladite composition étant sous la forme d'un comprimé.

4. Composition selon la revendication 1, ladite composition comprenant de l'acide alpha-lipoïque ou de l'alpha-tocophérol.

5. Composition selon la revendication 1, dans laquelle au moins a) 90 pour cent ; ou c) 95 pour cent de ladite préparation de polysaccharide de pomme de terre est du polysaccharide de pomme de terre.

6. Préparation de polysaccharide selon la revendication 1 obtenue à partir de pommes de terre crues destinée à être utilisée en :
a) augmentation de l'expression de polypeptides dans des cellules, lesdites cellules devant être mises en contact avec ladite préparation de polysaccharide de pomme de terre dans des conditions dans lesquelles l'expression d'un ou plusieurs des polypeptides choisis dans le groupe constitué par un polypeptide TFAM, un polypeptide ATP5A1, un polypeptide PDHA1, un polypeptide PDHA2 et un polypeptide THOP1 est augmentée ;
b) réduction de l'expression de polypeptides dans des cellules, lesdites cellules devant être mises en contact avec ladite préparation de polysaccharide de pomme de terre dans des conditions dans lesquelles l'expression d'un ou plusieurs des polypeptides choisis dans le groupe constitué par un polypeptide FOX01A, un polypeptide NFKB1, un polypeptide PDK2, un polypeptide PDK4 et un polypeptide HMGCR est réduite ; ou
c) traitement de l'obésité, du diabète et/ou du syndrome des ovaires polykystiques chez un mammifère,
(i) ledit mammifère devant être identifié comme ayant ladite obésité, ledit diabète et/ou ledit syndrome des ovaires polykystiques et
(ii) ladite préparation de polysaccharide de pomme de terre devant être administrée audit mammifère.
